# EUROPEAN PATENT APPLICATION

(11) **EP 4 545 516 A1**
(43) Date of publication of application: **30.04.2025**
(21) Application number: 25158535.2
(22) Date of filing: 01.06.2022
(51) Int. Cl.: C07D 211/38, C07D 211/52, C07D 401/10, C07D 401/14

(54) **PREPARATION OF A CHK1 INHIBITOR COMPOUND**

(30) Priority: 03.06.2021 GB 202107932
(62) Divisional of application: 22732474.6
(71) Applicant: Sentinel Oncology Limited, Cambridge Cambridgeshire CB4 0GJ (GB); Pharmaengine, Inc., Taiwan 10480 (TW)
(72) Inventor: Londesbrough, Derek John, Tyne & Wear, SR52TQ (GB); Chubb, Richard, Tyne & Wear, SR52TQ (GB); Travers, Stuart, Cambridge, CB4 0GJ (GB); Major, Meriel, Cambridge, CB4 0GJ (GB)
(74) Representative: Schlich

(57) **Abstract**

The invention provides a novel synthetic route for the preparation of the Chk-1 inhibitor compound: 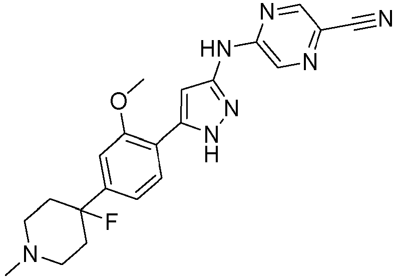 as well as novel process intermediates *per se.*

## Description

This invention relates to processes for preparing the Chk-1 inhibiitor compound 5-[[5-[4-(4-fluoro-1-methyl-4-piperidyl)-2-methoxy-phenyl]-1H-pyrazol-3-yl]amino]pyrazine-2-carbonitrile, processes for preparing synthetic intermediates, and to novel chemical intermediates for use in the processes.

### Background of the Invention

Chk-1 is a serine/threonine kinase involved in the induction of cell cycle checkpoints in response to DNA damage and replicative stress [Tse et al, Clin. Can. Res. 2007;13(7)]. Cell cycle checkpoints are regulatory pathways that control the order and timing of cell cycle transitions. Many cancer cells have impaired G1 checkpoint activation. For example, Hahn *et al.,* and Hollstein *et al.,* have reported that tumours are associated with mutations in the p53 gene, a tumour suppressor gene found in about 50% of all human cancers [N Engl J Med 2002, 347(20):1593; Science, 1991, 253(5015):49].

Chk-1 inhibition abrogates the intra S and G2/M checkpoints and has been shown to selectively sensitise tumour cells to well known DNA damaging agents. Examples of DNA damaging agents where this sensitising effect has been demonstrated include Gemcitabine, Pemetrexed, Cytarabine, Irinotecan, Camptothecin, Cisplatin, Carboplatin [Clin. Cancer Res. 2010, 16, 376], Temozolomide [Journal of Neurosurgery 2004, 100, 1060], Doxorubicin [Bioorg. Med. Chem. Lett. 2006;16:421-6], Paclitaxel [WO2010149394], Hydroxy urea [Nat. Cell. Biol. 2005;7(2):195-20], the nitroimidazole hypoxia-targetted drug TH-302 (Meng et al., AACR, 2013 Abstract No. 2389) and ionising radiation [Clin. Cancer Res. 2010, 16, 2076]*.* See also the review article by McNeely et al., [Pharmacology & Therapeutics (2014), 142(1):1-10]

Recently published data have also shown that Chk-1 inhibitors may act synergistically with PARP inhibitors [Cancer Res 2006.; 66:(16)], Mek inhibitors [Blood. 2008; 112(6): 2439-2449], Farnesyltransferase inhibitors [Blood. 2005;105(4):1706-16], Rapamycin [Mol. Cancer Ther. 2005;4(3):457-70], Src inhibitors [Blood. 2011;117(6):1947-57] and WEE1 inhibitors [Carrassa, 2021, 11(13):2507; Chaudhuri et al., Haematologica, 2014 99(4):688.].

Furthermore, Chk-1 inhibitors have demonstrated an advantage when combined with immunotherapy agents [Mouw et al., Br J Cancer, 2018. (7):933]. Chk1 inhibitors have been shown to activate cGAS, which induces an innate immune response through STING signaling, and to induce PD-L1 expression and synergize with anti-PD-L1 in vivo [Sen et al., Cancer Discov 2019 (5):646; Sen et al., J Thorac Oncol, 2019. (12):2152].

Resistance to chemotherapy and radiotherapy, a clinical problem for conventional therapy, has been associated with activation of the DNA damage response in which Chk-1 has been implicated [Nature; 2006; 444(7):756-760; Biochem. Biophys. Res. Commun. 2011 ;406(1):53-8].

It is also envisaged that Chk-1 inhibitors, either as single agents or in combination, may be useful in treating tumour cells in which constitutive activation of DNA damage and checkpoint pathways drive genomic instability in particular through replication stress. This phenotype is associated with complex karyotypes, for example in samples from patients with acute myeloid leukemia (AML) [Cancer Research 2009, 89, 8652]. *In vitro* antagonisation of the Chk-1 kinase with a small molecule inhibitor or by RNA interference strongly reduces the clonogenic properties of high-DNA damage level AML samples. In contrast Chk-1 inhibition has no effect on normal hematopoietic progenitors. Furthermore, recent studies have shown that the tumour microenvironment drives genetic instability [Nature; 2008;(8):180-192] and loss of Chk-1 sensitises cells to hypoxia/reoxygenation [Cell Cycle; 2010; 9(13):2502]. In neuroblastoma, a kinome RNA interference screen demonstrated that loss of Chk-1 inhibited the growth of eight neuroblastoma cell lines. Tumour cells deficient in Fanconi anemia DNA repair have shown sensitivity to Chk-1 inhibition [Molecular Cancer 2009, 8:24]. It has been shown that the Chk-1 specific inhibitor PF-00477736 inhibits the growth of thirty ovarian cancer cell lines [Bukczynska et al, 23rd Lorne Cancer Conference] and triple negative breast cancer cells [Cancer Science 2011, 102, 882]. Also, PF-00477736 has displayed selective single agent activity in a MYC oncogene driven murine spontaneous cancer model [Ferrao et al, Oncogene (15 August 2011)]. Chk-1 inhibition, by either RNA interference or selective small molecule inhibitors, results in apoptosis of MYC-overexpressing cells both *in vitro* and in an *in vivo* mouse model of B-cell lymphoma [Höglund et al., Clinical Cancer Research, 2011]. The latter data suggest that Chk-1 inhibitors would have utility for the treatment of MYC-driven malignancies such as B-cell lymphoma/leukemia, neuroblastoma and some breast and lung cancers. Chk-1 inhibitors have also been shown to be effective in pediatric tumour models, including Ewing's sarcoma and rhabdomyosarcoma [Lowery, 2018. Clin Cancer Res 2019, 25(7):2278]. Chk1 inhibitors have been shown to be synthetically lethal with the B-family of DNA polymerases, resulting in increased replication stress, DNA damage and cell death [Rogers et al., 2020, 80(8);1735]. Other cell cycle regulated genes have also been reported to confer sensitivity to Chk-1 inhibitors, including CDK2 and POXM1 [Ditano et al., 20201. 11(1);7077; Branigan et al., 2021 Cell Reports 34(9):1098808]

It has also been reported that mutations that reduce the activity of DNA repair pathways can result in synthetically lethal interactions with Chk1 inhibition. For example, mutations that disrupt the RAD50 complex and ATM signaling increase responsiveness to Chk1 inhibition [AI-Ahmadie et al., Cancer Discov. 2014. (9):1014-21]. Likewise, deficiencies in the Fanconi anemia homologous DNA repair pathway lead to sensitivity to Chk1 inhibition [Chen et al.,. Mol. Cancer 2009 8:24, Duan et al., Frontiers in Oncology 2014 4:368]. Also, human cells that have loss of function in the Rad17 gene product are sensitive to Chk1 suppression [Shen et al., Oncotarget, 2015. 6(34): 35755].

Various attempts have been made to develop inhibitors of Chk-1 kinase. For example, WO 03/10444 and WO 2005/072733 (both in the name of Millennium) disclose aryl/heteroaryl urea compounds as Chk-1 kinase inhibitors. US2005/215556 (Abbott) discloses macrocyclic ureas as kinase inhibitors. WO 02/070494, WO2006014359 and WO2006021002 (all in the name of Icos) disclose aryl and heteroaryl ureas as Chk-1 inhibitors. WO/2011/141716 and WO/2013/072502 both disclose substituted pyrazinyl-phenyl ureas as Chk-1 kinase inhibitors. WO2005/009435 (Pfizer) and WO2010/077758 (Eli Lilly) disclose aminopyrazoles as Chk-1 kinase inhibitors.

WO2015/120390 discloses a class of substituted phenyl-pyrazolyl-amines as Chk-1 kinase inhibitors. One of the compounds disclosed is the compound 5-[[5-[4-(4-fluoro-1-methyl-4-piperidyl)-2-methoxy-phenyl]-1H-pyrazol-3-yl]amino]pyrazine-2-carbonitrile, the synthesis of which is described in Example 64 and Synthetic Method L in WO2015/120390.

The Chk-1 kinase inhibitor compound 5-[[5-[4-(4-fluoro-1-methyl-4-piperidyl)-2-methoxy-phenyl]-1H-pyrazol-3-yl]amino]pyrazine-2-carbonitrile is useful in the treatment of cancers as disclosed in WO2015/120390.

WO2018/183891 (Cascadian Therapeutics) discloses combinations of the compound 5-[[5-[4-(4-fluoro-1-methyl-4-piperidyl)-2-methoxy-phenyl]-1H-pyrazol-3-yl]amino]pyrazine-2-carbonitrile or a pharmaceutically acceptable salt thereof with WEE-1 inhibitors.

### The Invention

The present invention provides improved processes for making the Chk-1 kinase inhibitor compound 5-[[5-[4-(4-fluoro-1-methyl-4-piperidyl)-2-methoxy-phenyl]-1H-pyrazol-3-yl]amino]pyrazine-2-carbonitrile (referred to herein also as the compound of formula (I) or the Chk-1 inhibitor).

In one general aspect, the improved process of the present invention is represented by the sequence of reactions set out in Scheme 1 below.

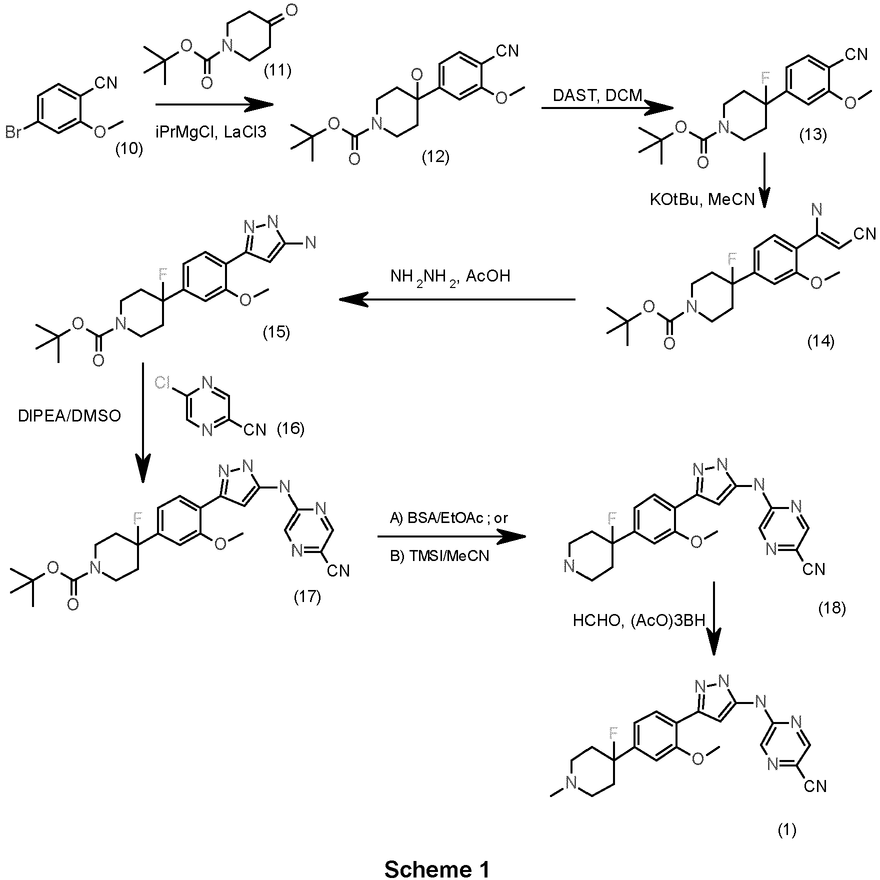

The synthetic route shown in Scheme 1 has a number of advantages over the synthetic route described in WO2015/120390. For example, the route depicted in Scheme 1 is shorter in terms of the total steps (7 vs 9). Many of the intermediates derived from the process are readily isolable crystalline solids. The new route therefore also makes use of these crystalline intermediates to remove the need for chromatography and thereby is a more scalable process. In addition, the improved process avoids the use of certain reagents in WO2015/120390 which are undesirable for large scale synthesis (e.g. Dess-Martin periodinane and n-BuLi).

The improved synthetic route makes use of the same final step (reductive methylation) as the synthetic route described in WO 2015/120390 but the deprotection and synthesis of the Boc-protected intermediate of formula (17) in the present route differ from the synthesis and deprotection of intermediate of formula (17) in WO2015/120390.

Accordingly, in one aspect (Embodiment 1.1), the invention provides a process for the preparation of a compound of the formula (18): 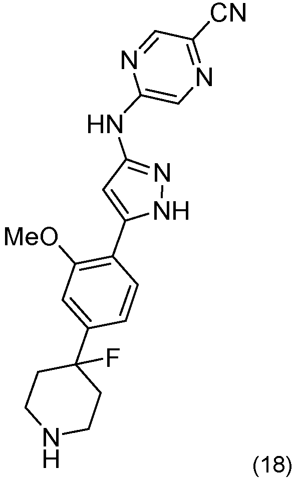 which process comprises reacting a compound of the formula (17): 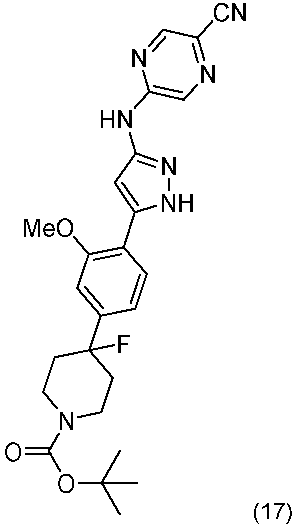 with an alkylsilyl halide or a sulphonic acid (such as benzenesulphonic acid).

The deprotection of Boc-protected intermediate of formula (17) in WO 2015/120390 made use of hydrochloric acid. However, it was found that residual amounts of hydrochloric acid present in the deprotected intermediate of formula (18) reacted with formaldehyde used in the subsequent reductive methylation in the reaction to form potential genotoxic impurities, for example the known carcinogen bis(chloromethyl) ether, (BCME). The improved process utilises either an alkylsilyl halide or benzenesulphonic acid (BSA) and has been found to avoid the generation of potential genotoxic impurities and other impurities in the final product. The process may further comprise reducing the water content of the starting material in order to give a cleaner deprotection step. However, the use of an alkylsilyl halide has been found to tolerate the presence of water in the starting material and therefore an advantage of using an alkyl alkylsilyl halide over BSA is that it avoids the need for a pre-reaction drying step. The use of alkylsilyl halide also reduces the reaction temperature and reaction time and avoids the use of reagents that may contain benzene impurities.

In further embodiments, the invention provides:
1.2 A process according to Embodiment 1.1 comprising reacting a compound of the formula (17) with an alkylsilyl halide.
1.3 A process according to Embodiment 1.2 wherein the alkylsilyl halide is a trimethyl silyl halide.
1.4 A process according to Embodiment 1.3 wherein the alkylsilyl halide is trimethylsilyl iodide (TMSI).
1.5 A process according to any one of Embodiments 1.1 to 1.4 wherein the reaction is carried out in the presence of a polar, aprotic solvent.
1.6 A process according to Embodiment 1.5 wherein the polar, aprotic solvent is acetonitrile.
1.7 A process according to any one of Embodiments 1.1 to 1.6 wherein the reaction is carried out at a temperature of from 0 °C to 20 °C.
1.8 A process according to any one of Embodiments 1.1 to 1.7 wherein the reaction is carried out for a period of from 15 minutes to 60 minutes, for example for a
period of approximately 30 minutes and is optionally followed by the addition of a base (such as an alkali metal carbonate, e.g. potassium carbonate).
1.9 A process according to Embodiment 1.1 comprising reacting a compound of the formula (17) with a sulphonic acid, such as benzenesulphonic acid.
1.10 A process according to Embodiment 1.9 wherein the reaction is carried out in the presence of a polar, aprotic solvent.
1.11 A process according to Embodiment 1.10 wherein the polar, aprotic solvent is ethyl acetate.
1.12 A process according to any one of Embodiments 1.9 to 1.11 wherein the reaction is carried out at a temperature of from 50 °C to 70 °C, for example at a temperature of approximately 60 °C.
1.13 A process according to any one of Embodiments 1.9 to 1.11 wherein the reaction is carried out for a period of from 20 hours to 30 hours, for example for a period of approximately 26 hours.
1.14 A process according to any one of Embodiments 1.9 to 1.11 which, prior to reacting the compound of formula (17) with benzenesulphonic acid, comprises the step of reducing the water content of the starting material.
1.15 A process according to Embodiment 1.14 wherein the step of reducing the water content of the starting material involves heating the compound of formula (17) in a non-polar solvent (such as 1,4-dioxane), for example at a temperature of from 80 °C to 85 °C, and then removing the non-polar solvent.

In another aspect (Embodiment 2.1) the invention provides a process for the preparation of a compound of the formula (17): 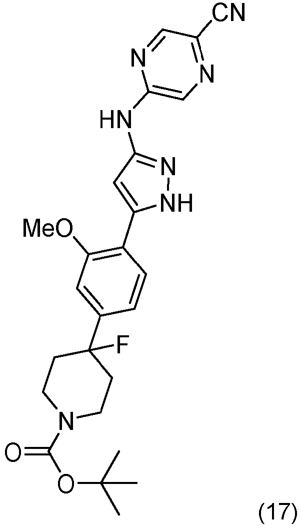 which process comprises the reaction of a compound of the formula (15) 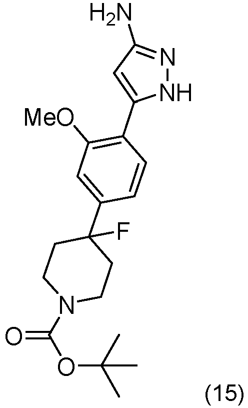 with a compound of the formula (16):

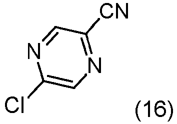

In WO 2015/120390, Boc-protected intermediate of formula (17) was formed by the reaction of intermediate of formula (15) with 5-bromopyrazine-2-carbonitrile. By contrast, the improved process makes use of 5-chloropyrazine-2-carbonitrile. In addition, in the improved process, the reaction is conducted at a lower temperature than described in WO 2015/120390 (50°C rather than 80°C).

Furthermore, it has been found that conducting the reaction in anhydrous conditions reduces the formation of unwanted side-products/impurities.

In further embodiments, the invention provides:
2.2 A process according to Embodiment 2.1 wherein the reaction is carried out in the reaction is carried out in a non-aqueous aprotic solvent.
2.3 A process according to Embodiment 2.2 wherein the solvent is dimethyl sulphoxide (DMSO).
2.4 A process according to any one of Embodiments 2.1 to 2.3 wherein the solvent is anhydrous, for example wherein the solvent has a water content of 500ppm or less.
2.5 A process according to any one of Embodiments 2.1 to 2.3 wherein the reaction is carried out in the presence of diisopropylethylamine (DIPEA).
2.6 A process according to any one of Embodiments 2.1 to 2.5 wherein the process is carried out at a temperature in the range from 40 °C to 80 °C, e.g. about 70 °C.

In the improved process, the method of preparing intermediate of formula (15) is entirely different to the method used in WO 2015/120390. The improved method results in a number of advantages described below, including:
- The use of crystalline intermediates (which reduces the need for chromatographic purification of intermediates)
- The lowest temperature used is -10 °C, making the process more suitable for scaling (the route described in WO 2015/120390 involved two reaction steps that were conducted at -78 °C)
- The reactions being higher yielding
- Avoiding the use of Dess-Martin periodinane as an oxidizing reagent. Use of this reagent on an industrial scale is made difficult by its cost and its potentially explosive nature (see Plumb, J.B.; Harper, D.J. (1990). "Chemical Safety: 2-lodoxybenzoic acid". Chem. Eng. News. 68: 3. doi:10.1021/cen-v068n029.p002)

Further specific advantages are discussed in relation to each step below.

In a further aspect (Embodiment 3.1), the invention provides a process for the preparation of a compound of formula (15): 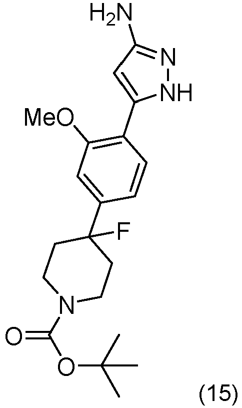 which process comprises reacting a compound of the formula (14): 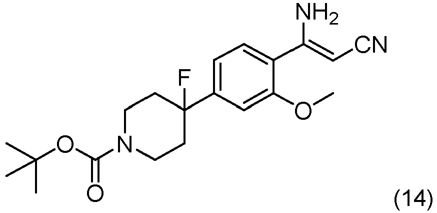 with hydrazine (NH₂NH₂).

This process results in an improved yield compared to the corresponding process for the formation of a compound of formula (15) in WO 2015/120390 (see Example 64). The improved process results in a yield of 58% compared to a yield of 44% obtained with the process of Example 64.

In further embodiments, the invention provides:
3.2 A process according to Embodiment 3.1 wherein the reaction is carried out in a polar, protic solvent.
3.3 A process according to Embodiment 3.2 wherein the polar, protic solvent is a C₁₋₄ alcohol, such as ethanol (EtOH).
3.4 A process according to any one of Embodiments 3.1 to 3.3 wherein the reaction is carried out in the present of glacial acetic acid.
3.5 A process according to any one of Embodiment 3.1 to 3.4 wherein the reaction is carried out at a temperature of 70 °C to 80 °C, for example at a temperature of approximately 75 °C.
3.6 A process according to any one of Embodiments 3.1 to 3.5 wherein the reaction is carried out for a time period of 4 hours or less, for example from 1 hour to 3 hours, such as for approximately 2 hours.

In a further aspect (Embodiment 4.1), the invention provides a process for the preparation of a compound of the formula (14): 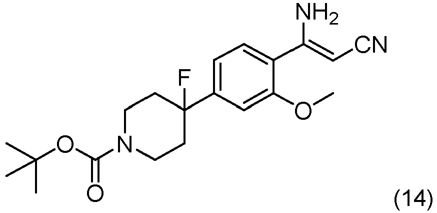 which process comprises reacting a compound of formula (13): 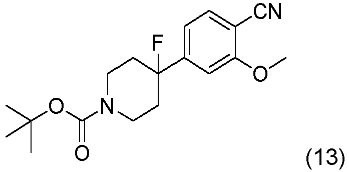 with acetonitrile (CH₃CN) in the presence of a base.

In further embodiments, the invention provides:
4.2 A process according to Embodiment 4.1 wherein the base is an alkoxide base.
4.3 A process according to Embodiment 4.2 wherein the base is a tert-butoxide base.
4.4 A process according to Embodiment 4.3 wherein the base is KO^{t}Bu.
4.5 A process according to any one of Embodiments 4.1 to 4.4 wherein the reaction is carried out in a polar, aprotic solvent.
4.6 A process according to Embodiment 4.5 wherein the solvent is tetrahydrofuran (THF) or diethyl ether.
4.7 A process according to any one of Embodiment 4.1 to 4.6 wherein the reaction is carried out at a temperature of 30 °C to 50 °C, for example at a temperature of approximately 40 °C.
4.8 A process according to any one of Embodiments 4.1 to 4.7 wherein the reaction is carried out for a time period of from 3 hours to 5 hours, for example for a time period of approximately 4 hours.

The compound of formula (13) is prepared by the fluorination of the corresponding alcohol compound of formula (12).

Accordingly, in a further aspect (Embodiment 5.1), the invention provides a process for the preparation of a compound of the formula (13): 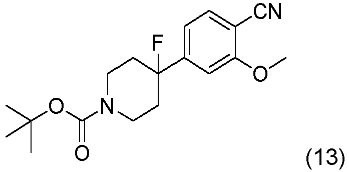 which process comprises reacting a compound of formula (12): 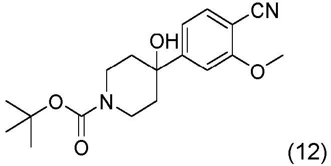 with a deoxyfluorination reagent.

In further embodiments, the invention provides:
5.2 A process according to Embodiment 5.1 wherein the deoxyfluorination reagent is diethylaminosulphur trifluoride DAST.
5.3 A process according to Embodiment 5.1 or Embodiment 5.2 wherein the reaction is carried out in a polar, aprotic solvent.
5.4 A process according to Embodiment 5.3 wherein the solvent is dichloromethane (DCM).
5.5 A process according to any one of Embodiments 5.1 to 5.4 wherein the reaction is carried out at a temperature of between -20 °C and -10°C (for example, at approximately -10°C).

In a further aspect (Embodiment 6.1), the invention provides a process for the preparation of a compound of the formula (12): 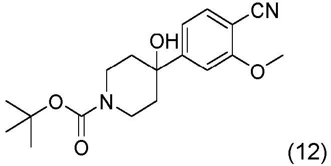 which process comprises reacting a compound of formula (10): 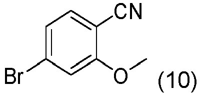 with a compound of formula (11):

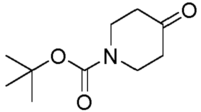

in the presence of a metallating agent, such as a Grignard reagent.

The reaction is typically carried out in the presence of a Grignard reagent and a Lewis acid, for example LaCl₃. It has been found that the LaCl₃ reagent acts as a chelating reagent and reduces the formation of impurities.

Accordingly, in further embodiments, the invention provides:
6.2 A process according to Embodiment 6.1 wherein the reaction is carried out in the presence of a compound of the formula RMgCl, wherein R is a C₁₋₄ alkyl group.
6.3 A process according to Embodiment 6.2 wherein R is iso-propyl (and RMgCl is iPrMgCl).
6.4 A process according to any one of Embodiments 6.1 to 6.3 wherein the reaction is carried out in the presence of a Lewis acid.
6.5 A process according to Embodiment 6.4 wherein the Lewis acid is a lanthanide chloride (e.g. LaCl₃ or CeCl₃).
6.6 A process according to Embodiment 6.4 or 6.5 wherein the Lewis acid is LaCl₃.
6.7 A process according to any one of Embodiments 6.1 to 6.6 wherein the reaction is carried out in a polar, aprotic solvent.
6.8 A process according to Embodiment 6.7 wherein the solvent is tetrahydrofuran (THF).

The above processes can be used in the preparation of 5-[[5-[4-(4-fluoro-1-methyl-4-piperidyl)-2-methoxy-phenyl]-1H-pyrazol-3-yl]amino]pyrazine-2-carbonitrile or pharmaceutically acceptable salts thereof. Accordingly, in a further aspect, there is provided a process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
i)
   a) a process according to any one of Embodiments 6.1 to 6.8; and/or
   b) a process according to any one of Embodiment 5.1 to 5.5; and/or
   c) a process according to any one of Embodiment 4.1 to 4.8; and/or
   d) a process according to any one of Embodiment 3.1 to 3.6; and/or
   e) a process according to any one of Embodiment 2.1 to 2.6; and/or
   f) a process according to any one of Embodiment 1.1 to 1.7; and
ii) interconverting a product obtained from step i) into 5-[[5-[4-(4-fluoro-1-methyl-4-piperidyl)-2-methoxy-phenyl]-1H-pyrazol-3-yl]amino]pyrazine-2-carbonitrile, for example by reacting a compound of formula (18) with a methylating agent (such as HCHO in the presence of a reducing agent such as (AcO₃)BH); and
iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile. Alternatively, the invention provides a process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
   i)
      a) a process according to Embodiment 8.4; and/or
      b) a process according to Embodiment 8.3; and/or
      c) a process according to Embodiment 8.2; and/or
      d) a process according to Embodiment 8.1;
   ii) interconverting a product obtained from step i) to a compound of formula (18);
   iii) interconverting a product obtained from step ii) into 5-[[5-[4-(4-fluoro-1-methyl-4-piperidyl)-2-methoxy-phenyl]-1H-pyrazol-3-yl]amino]pyrazine-2-carbonitrile, for example by reacting a compound of formula (18) with a methylating agent (such as HCHO in the presence of a reducing agentsuch as (AcO₃)BH); and
   iv) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

In a further aspect, the invention provides novel intermediates suitable for use in the processes of the invention. It has been found that the intermediates are crystalline and therefore the need for chromatography to purify reaction products is reduced or avoided.

Accordingly, in further embodiments, the invention provides:
7.1 A compound of the formula (14): 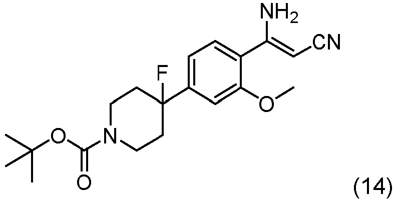
7.2 A compound of the formula (13): 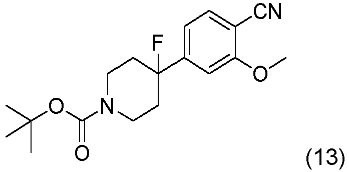
7.3 A compound of the formula (12): 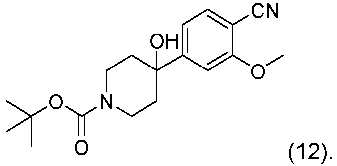
7.4 A compound according to any one of Embodiments 7.1 to 7.3 in a substantially crystalline form.

Further details of crystalline forms of the intermediates used in the improved process are provided below.

### Intermediate of Formula (12)

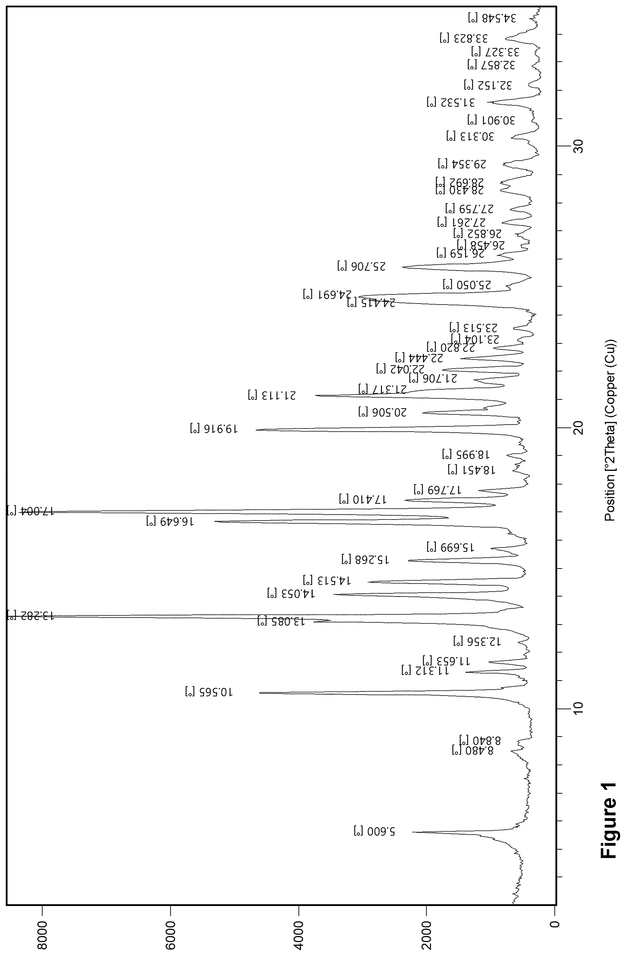

The XRPD diffractogram for a crystalline form of the intermediate of formula (12) is shown in Figure 1.

The X-ray diffraction pattern of the crystalline form of the intermediate of formula (12) exhibits peaks of greatest intensity at the diffraction angles (2θ) set out in Table A-1

| Table A-1 | |
|---|---|
| **Diffraction Angle (°)** | **Relative Intensity** |
| 10.6 ± 0.2 | 54 |
| 13.3 ± 0.2 | 100 |
| 16.7 ± 0.2 | 61 |
| 17.0 ± 0.2 | 99 |
| 19.9 ± 0.2 | 52 |

Accordingly, in further embodiments, the invention provides:
7.5 A substantially crystalline form of the compound of formula (12) having an X-ray powder diffraction pattern characterised by the presence of major peaks at the diffraction angles (2θ) 10.6° and/or 13.3° and/or 16.7° and/or 17.0° and/or 19.9° (±0.2°).
7.6 A substantially crystalline form of the compound of formula (12) having an X-ray powder diffraction pattern characterised by the presence of a major peak at the diffraction angle (2θ) 10.6° (±0.2°).
7.7 A substantially crystalline form of the compound of formula (12) having an X-ray powder diffraction pattern characterised by the presence of a major peak at the diffraction angle (2θ) 13.3° (±0.2°).
7.8 A substantially crystalline form of the compound of formula (12) having an X-ray powder diffraction pattern characterised by the presence of a major peak at the diffraction angle (2θ) 16.7° (±0.2°).
7.9 A substantially crystalline form of the compound of formula (12) having an X-ray powder diffraction pattern characterised by the presence of a major peak at the diffraction angle (2θ) 17.0° (±0.2°).
7.10 A substantially crystalline form of the compound of formula (12) having an X-ray powder diffraction pattern characterised by the presence of a major peak at the diffraction angle (2θ) 19.9° (±0.2°).
7.11 A substantially crystalline form of the compound of formula (12) having an X-ray powder diffraction pattern characterised by the presence of major peaks at two or more, e.g. three or more, or four or more, and in particular five diffraction angles (2θ) selected from 10.6°, 13.3°, 16.7°, 17.0° and 19.9° (±0.2°).
7.12 A substantially crystalline form of the compound of formula (12) which exhibits peaks at the diffraction angles set forth in Table A-1 provided above or Table 1 provided below which have a relative intensity of at least 15%.
7.13 A substantially crystalline form of the compound of formula (12) which exhibits peaks at the diffraction angles corresponding to those of the X-ray powder diffraction pattern shown in Figure 1.
7.14 A substantially crystalline form of the compound of formula (12) having an X-ray powder diffraction pattern substantially as shown in Figure 1.

### Intermediate of Formula (13)

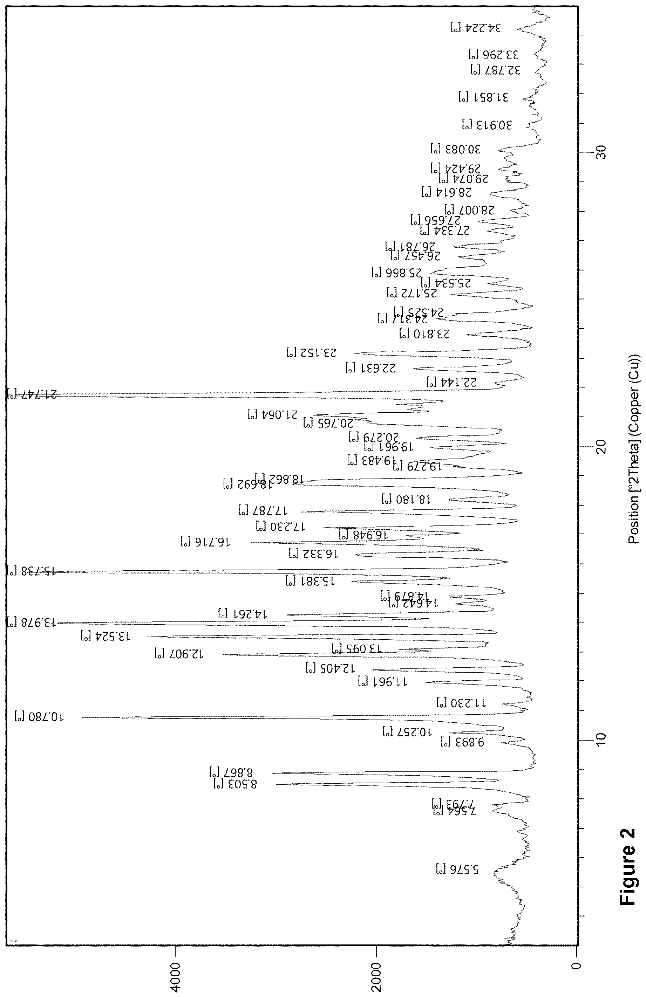

The XRPD diffractogram for a crystalline form of the intermediate of formula (13) is shown in Figure 2.

The X-ray diffraction pattern of the crystalline form of the intermediate of formula (13) exhibits peaks of greatest intensity at the diffraction angles (2θ) set out in Table A-2

| Table A-2 | |
|---|---|
| **Diffraction Angle (°)** | **Relative Intensity** |
| 10.8 ± 0.2 | 87 |
| 13.5 ± 0.2 | 71 |
| 14.0 ± 0.2 | 89 |
| 15.7 ± 0.2 | 91 |
| 21.7 ± 0.2 | 100 |

Accordingly, in further embodiments, the invention provides:
7.15 A substantially crystalline form of the compound of formula (13) having an X-ray powder diffraction pattern characterised by the presence of major peaks at the diffraction angles (2θ) 10.8° and/or 13.5° and/or 14.0° and/or 15.7° and/or 21.7° (±0.2°).
7.16 A substantially crystalline form of the compound of formula (13) having an X-ray powder diffraction pattern characterised by the presence of a major peak at the diffraction angle (2θ) 10.8° (±0.2°).
7.17 A substantially crystalline form of the compound of formula (13) having an X-ray powder diffraction pattern characterised by the presence of a major peak at the diffraction angle (2θ) 13.5° (±0.2°).
7.18 A substantially crystalline form of the compound of formula (13) having an X-ray powder diffraction pattern characterised by the presence of a major peak at the diffraction angle (2θ) 14.0° (±0.2°).
7.19 A substantially crystalline form of the compound of formula (13) having an X-ray powder diffraction pattern characterised by the presence of a major peak at the diffraction angle (2θ) 15.7° (±0.2°).
7.20 A substantially crystalline form of the compound of formula (13) having an X-ray powder diffraction pattern characterised by the presence of a major peak at the diffraction angle (2θ) 21.7° (±0.2°).
7.21 A substantially crystalline form of the compound of formula (13) having an X-ray powder diffraction pattern characterised by the presence of major peaks at two or more, e.g. three or more, or four or more, and in particular five diffraction angles (2θ) selected from 10.8°, 13.5°, 14.0°, 15.7° and 21.7° (±0.2°).
7.22 A substantially crystalline form of the compound of formula (13) which exhibits peaks at the diffraction angles set forth in Table A-2 provided above or Table 2 provided below which have a relative intensity of at least 15%.
7.23 A substantially crystalline form of the compound of formula (13) which exhibits peaks at the diffraction angles corresponding to those of the X-ray powder diffraction pattern shown in Figure 2.
7.24 A substantially crystalline form of the compound of formula (13) having an X-ray powder diffraction pattern substantially as shown in Figure 2.

### Intermediate of Formula (14)

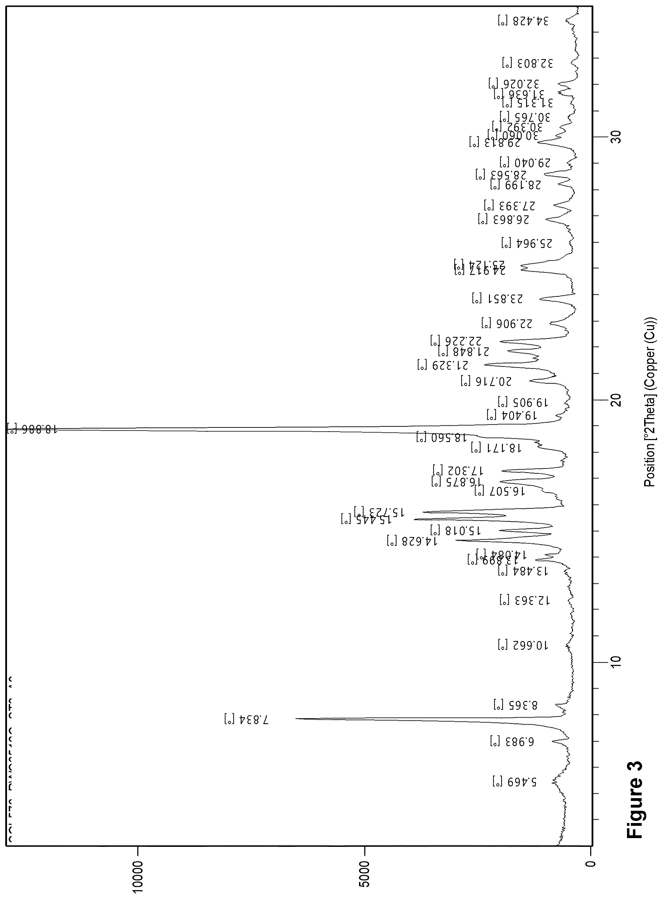

The XRPD diffractogram for a crystalline form of the intermediate of formula (14) is shown in Figure 3.

The X-ray diffraction pattern of the crystalline form of the intermediate of formula (14) exhibits peaks of greatest intensity at the diffraction angles (2θ) set out in Table A-3

| Table A-3 | |
|---|---|
| **Diffraction Angle (°)** | **Relative Intensity** |
| 7.8 ± 0.2 | 50 |
| 14.6 ± 0.2 | 19 |
| 15.4 ± 0.2 | 26 |
| 15.7 ± 0.2 | 24 |
| 18.9 ± 0.2 | 100 |

Accordingly, in further embodiments, the invention provides:
7.25 A substantially crystalline form of the compound of formula (14) having an X-ray powder diffraction pattern characterised by the presence of major peaks at the diffraction angles (2θ) 7.8° and/or 14.6° and/or 15.4° and/or 15.7° and/or 18.9° (±0.2°).
7.26 A substantially crystalline form of the compound of formula (14) having an X-ray powder diffraction pattern characterised by the presence of a major peak at the diffraction angle (2θ) 7.8° (±0.2°).
7.27 A substantially crystalline form of the compound of formula (14) having an X-ray powder diffraction pattern characterised by the presence of a major peak at the diffraction angle (2θ) 14.6° (±0.2°).
7.28 A substantially crystalline form of the compound of formula (14) having an X-ray powder diffraction pattern characterised by the presence of a major peak at the diffraction angle (2θ) 15.4° (±0.2°).
7.29 A substantially crystalline form of the compound of formula (14) having an X-ray powder diffraction pattern characterised by the presence of a major peak at the diffraction angle (2θ) 15.7° (±0.2°).
7.30 A substantially crystalline form of the compound of formula (14) having an X-ray powder diffraction pattern characterised by the presence of a major peak at the diffraction angle (2θ) 18.9° (±0.2°).
7.31 A substantially crystalline form of the compound of formula (14) having an X-ray powder diffraction pattern characterised by the presence of major peaks at two or more, e.g. three or more, or four or more, and in particular five diffraction angles (2θ) selected from 10.7°, 14.6°, 15.4°, 15.7° and 18.9° (±0.2°).
7.32 A substantially crystalline form of the compound of formula (14) which exhibits peaks at the diffraction angles set forth in Table A-3 provided above or Table 3 provided below which have a relative intensity of at least 15%.
7.33 A substantially crystalline form of the compound of formula (14) which exhibits peaks at the diffraction angles corresponding to those of the X-ray powder diffraction pattern shown in Figure 3.
7.34 A substantially crystalline form of the compound of formula (14) having an X-ray powder diffraction pattern substantially as shown in Figure 3.

### Intermediate of Formula (15)

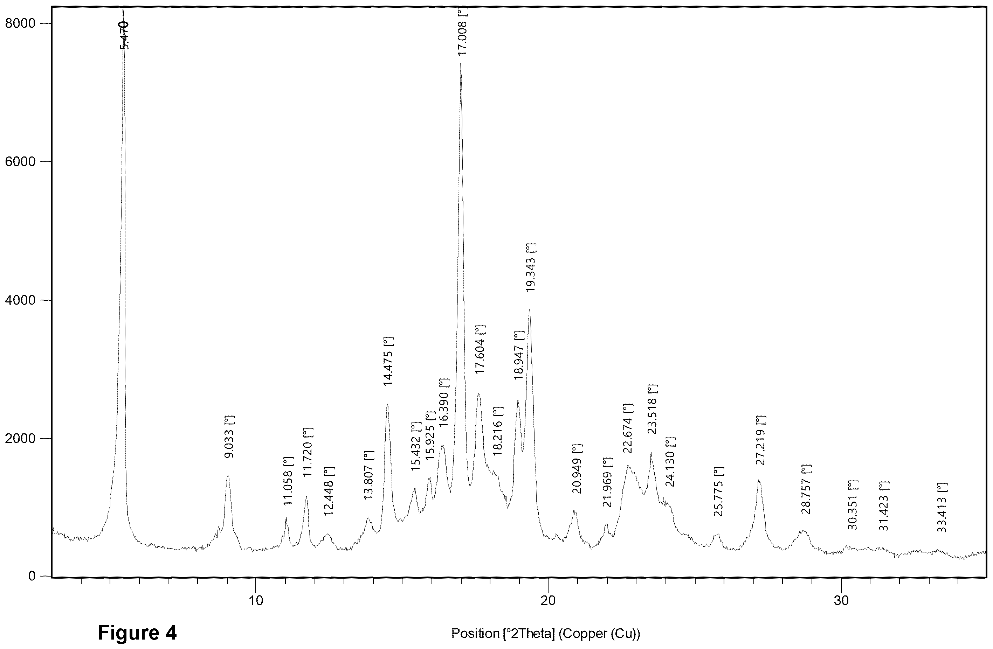

The XRPD diffractogram for a crystalline form of intermediate of formula (15) is shown in Figure 4.

The X-ray diffraction pattern of the crystalline form of intermediate of formula (15) exhibits peaks of greatest intensity at the diffraction angles (2θ) set out in Table A-4

| Table A-4 | |
|---|---|
| **Diffraction Angle (°)** | **Relative Intensity** |
| 5.5 ± 0.2 | 100 |
| 14.5 ± 0.2 | 22 |
| 17.0 ± 0.2 | 80 |
| 18.9 ± 0.2 | 22 |
| 19.3 ± 0.2 | 39 |

Accordingly, in further embodiments, the invention provides:
7.35 A compound of formula (15) in a substantially crystalline form.
7.36 A substantially crystalline form of the compound of formula (15) having an X-ray powder diffraction pattern characterised by the presence of major peaks at the diffraction angles (2θ) 5.5° and/or 14.5° and/or 17.0° and/or 18.9° and/or 19.3° (±0.2°).
7.37 A substantially crystalline form of the compound of formula (15) having an X-ray powder diffraction pattern characterised by the presence of a major peak at the diffraction angle (2θ) 5.5° (±0.2°).
7.38 A substantially crystalline form of the compound of formula (15) having an X-ray powder diffraction pattern characterised by the presence of a major peak at the diffraction angle (2θ) 14.5° (±0.2°).
7.39 A substantially crystalline form of the compound of formula (15) having an X-ray powder diffraction pattern characterised by the presence of a major peak at the diffraction angle (2θ) 17.0° (±0.2°).
7.40 A substantially crystalline form of the compound of formula (15) having an X-ray powder diffraction pattern characterised by the presence of a major peak at the diffraction angle (2θ) 18.9° (±0.2°).
7.41 A substantially crystalline form of the compound of formula (15) having an X-ray powder diffraction pattern characterised by the presence of a major peak at the diffraction angle (2θ) 19.3° (±0.2°).
7.42 A substantially crystalline form of the compound of formula (15) having an X-ray powder diffraction pattern characterised by the presence of major peaks at two or more, e.g. three or more, or four or more, and in particular five diffraction angles (2θ) selected from 5.5°, 14.5°, 17.0°, 18.9° and 19.3° (±0.2°).
7.43 A substantially crystalline form of the compound of formula (15) which exhibits peaks at the diffraction angles set forth in Table A-4 provided above or Table 4 provided below which have a relative intensity of at least 15%.
7.44 A substantially crystalline form of the compound of formula (15) which exhibits peaks at the diffraction angles corresponding to those of the X-ray powder diffraction pattern shown in Figure 4.
7.45 A substantially crystalline form of the compound of formula (15) having an X-ray powder diffraction pattern substantially as shown in Figure 4.

### Intermediate of Formula (17)

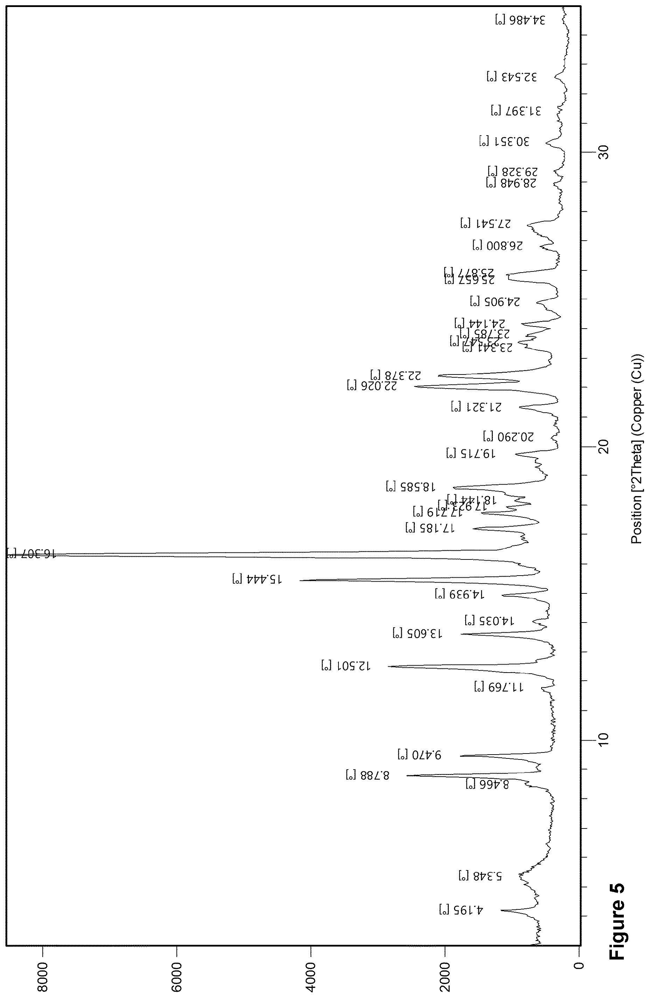

The XRPD diffractogram for a crystalline form of the intermediate of formula (17) is shown in Figure 5.

The X-ray diffraction pattern of the crystalline form of the intermediate of formula (17) exhibits peaks of greatest intensity at the diffraction angles (2θ) set out in Table A-5

| Table A-5 | |
|---|---|
| **Diffraction Angle (°)** | **Relative Intensity** |
| 8.8 ± 0.2 | 27 |
| 12.5 ± 0.2 | 30 |
| 15.4 ± 0.2 | 45 |
| 16.3 ± 0.2 | 100 |
| 22.0 ± 0.2 | 26 |

Accordingly, in further embodiments, the invention provides:
7.46 A compound of formula (17) in a substantially crystalline form.
7.47 A substantially crystalline form of the compound of formula (17) having an X-ray powder diffraction pattern characterised by the presence of major peaks at the diffraction angles (2θ) 8.8° and/or 12.5° and/or 15.4° and/or 16.3° and/or 22.0° (±0.2°).
7.48 A substantially crystalline form of the compound of formula (17) having an X-ray powder diffraction pattern characterised by the presence of a major peak at the diffraction angle (2θ) 8.8° (±0.2°).
7.49 A substantially crystalline form of the compound of formula (17) having an X-ray powder diffraction pattern characterised by the presence of a major peak at the diffraction angle (2θ) 12.5° (±0.2°).
7.50 A substantially crystalline form of the compound of formula (17) having an X-ray powder diffraction pattern characterised by the presence of a major peak at the diffraction angle (2θ) 15.4° (±0.2°).
7.51 A substantially crystalline form of the compound of formula (17) having an X-ray powder diffraction pattern characterised by the presence of a major peak at the diffraction angle (2θ) 16.3° (±0.2°).
7.52 A substantially crystalline form of the compound of formula (17) having an X-ray powder diffraction pattern characterised by the presence of a major peak at the diffraction angle (2θ) 22.0° (±0.2°).
7.53 A substantially crystalline form of the compound of formula (17) having an X-ray powder diffraction pattern characterised by the presence of major peaks at two or more, e.g. three or more, or four or more, and in particular five diffraction angles (2θ) selected from 8.8°, 12.5°, 15.4°, 16.3° and 22.0° (±0.2°).
7.54 A substantially crystalline form of the compound of formula (17) which exhibits peaks at the diffraction angles set forth in Table A-5 provided above or Table 5 provided below which have a relative intensity of at least 15%.
7.55 A substantially crystalline form of the compound of formula (17) which exhibits peaks at the diffraction angles corresponding to those of the X-ray powder diffraction pattern shown in Figure 5.
7.56 A substantially crystalline form of the compound of formula (17) having an X-ray powder diffraction pattern substantially as shown in Figure 5.

### Intermediate of Formula (18)

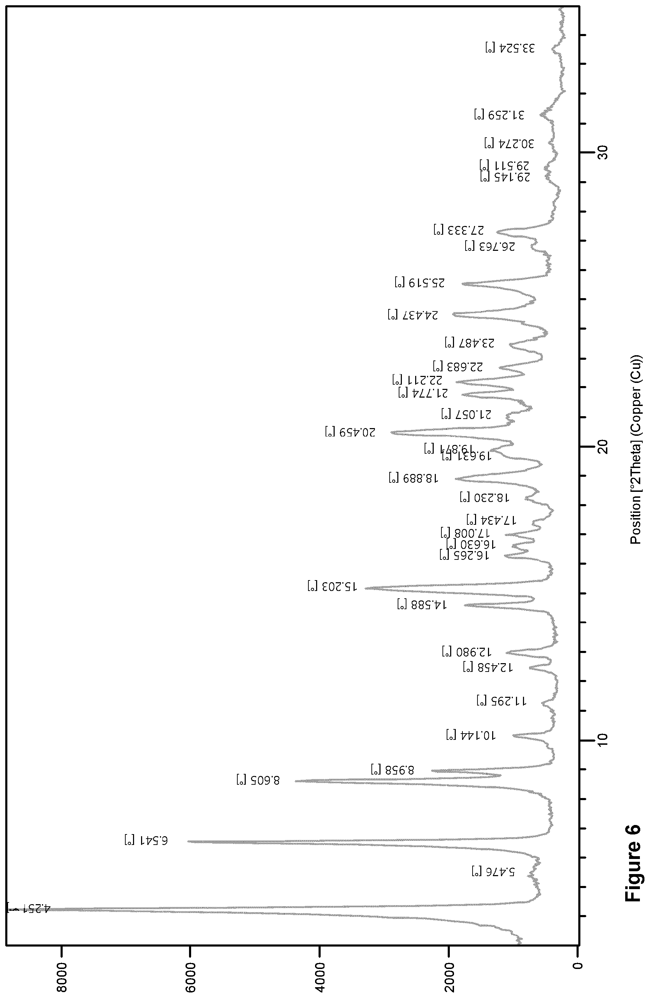

The XRPD diffractogram for a crystalline form of the benzenesulphonate salt of the intermediate of formula (18) is shown in Figure 6.

The X-ray diffraction pattern of the crystalline form of the benzenesulphonate salt of the intermediate of formula (18) exhibits peaks of greatest intensity at the diffraction angles (2θ) set out in Table A-6

| Table A-6 | |
|---|---|
| **Diffraction Angle (°)** | **Relative Intensity** |
| 4.3 ± 0.2 | 100 |
| 6.5 ± 0.2 | 68 |
| 8.6 ± 0.2 | 48 |
| 15.2 ± 0.2 | 33 |
| 20.5 ± 0.2 | 26 |

Accordingly, in further embodiments, the invention provides:
7.57 A benzenesulphonate salt of a compound of formula (18) in a substantially crystalline form.
7.58 A substantially crystalline form of a benzenesulphonate salt of the compound of formula (18) having an X-ray powder diffraction pattern characterised by the presence of major peaks at the diffraction angles (2θ) 4.3° and/or 6.5° and/or 8.6° and/or 15.2° and/or 20.5° (±0.2°).
7.59 A substantially crystalline form of a benzenesulphonate salt of the compound of formula (18) having an X-ray powder diffraction pattern characterised by the presence of a major peak at the diffraction angle (2θ) 4.3° (±0.2°).
7.60 A substantially crystalline form of a benzenesulphonate salt of the compound of formula (18) having an X-ray powder diffraction pattern characterised by the presence of a major peak at the diffraction angle (2θ) 6.5° (±0.2°).
7.61 A substantially crystalline form of a benzenesulphonate salt of the compound of formula (18) having an X-ray powder diffraction pattern characterised by the presence of a major peak at the diffraction angle (2θ) 8.6° (±0.2°).
7.62 A substantially crystalline form of a benzenesulphonate salt of the compound of formula (18) having an X-ray powder diffraction pattern characterised by the presence of a major peak at the diffraction angle (2θ) 15.2° (±0.2°).
7.63 A substantially crystalline form of a benzenesulphonate salt of the compound of formula (18) having an X-ray powder diffraction pattern characterised by the presence of a major peak at the diffraction angle (2θ) 20.5° (±0.2°).
7.64 A substantially crystalline form of a benzenesulphonate salt of the compound of formula (18) having an X-ray powder diffraction pattern characterised by the presence of major peaks at two or more, e.g. three or more, or four or more, and in particular five diffraction angles (2θ) selected from 4.3°, 6.5°, 8.6°, 15.2° and 20.5° (±0.2°).
7.65 A substantially crystalline form of a benzenesulphonate salt of the compound of formula (18) which exhibits peaks at the diffraction angles set forth in Table A-6 provided above or Table 6 provided below which have a relative intensity of at least 15%.
7.66 A substantially crystalline form of a benzenesulphonate salt of the compound of formula (18) which exhibits peaks at the diffraction angles corresponding to those of the X-ray powder diffraction pattern shown in Figure 6.
7.67 A substantially crystalline form of a benzenesulphonate salt of the compound of formula (18) having an X-ray powder diffraction pattern substantially as shown in Figure 6.

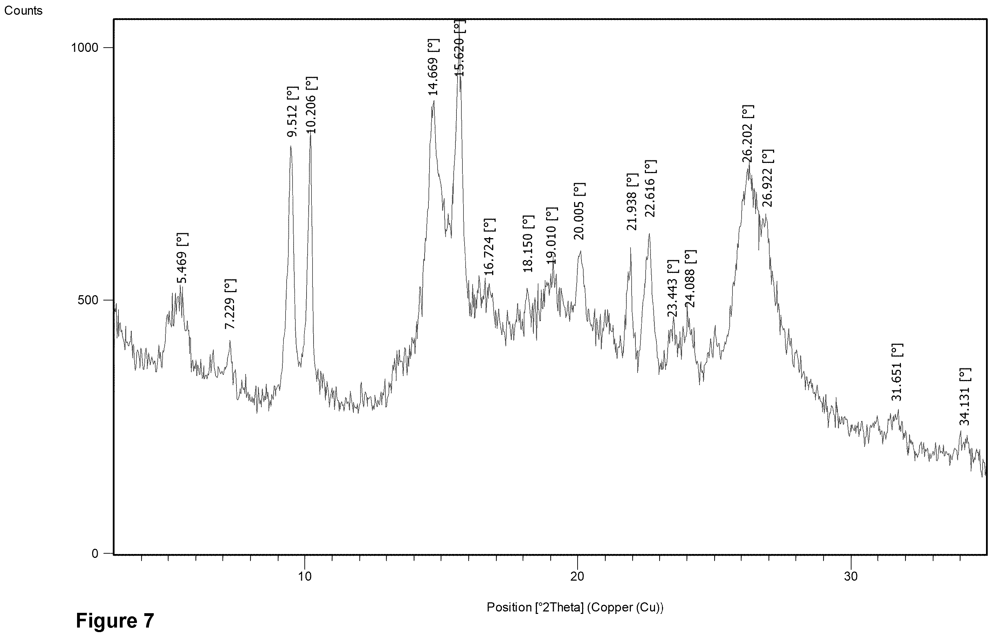

The XRPD diffractogram for a crystalline form of the free base of the intermediate of formula (18) is shown in Figure 7.

The X-ray diffraction pattern of the crystalline form of the intermediate of formula (18) exhibits peaks of greatest intensity at the diffraction angles (2θ) set out in Table A-7

| Table A-7 | |
|---|---|
| **Diffraction Angle (°)** | **Relative Intensity** |
| 9.5 ± 0.2 | 85 |
| 10.2 ± 0.2 | 87 |
| 14.7 ± 0.2 | 86 |
| 15.6 ± 0.2 | 100 |
| 26.2 ± 0.2 | 67 |

Accordingly, in further embodiments, the invention provides:
7.68 A compound of formula (18) in a substantially crystalline form.
7.69 A substantially crystalline form of a compound of formula (18) having an X-ray powder diffraction pattern characterised by the presence of major peaks at the diffraction angles (2θ) 9.5° and/or 10.2° and/or 14.7° and/or 15.6° and/or 26.2° (±0.2°).
7.70 A substantially crystalline form of a compound of formula (18) having an X-ray powder diffraction pattern characterised by the presence of a major peak at the diffraction angle (2θ) 9.5° (±0.2°).
7.71 A substantially crystalline form of a compound of formula (18) having an X-ray powder diffraction pattern characterised by the presence of a major peak at the diffraction angle (2θ) 10.2° (±0.2°).
7.72 A substantially crystalline form of a compound of formula (18) having an X-ray powder diffraction pattern characterised by the presence of a major peak at the diffraction angle (2θ) 14.7° (±0.2°).
7.73 A substantially crystalline form of a compound of formula (18) having an X-ray powder diffraction pattern characterised by the presence of a major peak at the diffraction angle (2θ) 15.6° (±0.2°).
7.74 A substantially crystalline form of a compound of formula (18) having an X-ray powder diffraction pattern characterised by the presence of a major peak at the diffraction angle (2θ) 26.2° (±0.2°).
7.75 A substantially crystalline form of a compound of formula (18) having an X-ray powder diffraction pattern characterised by the presence of major peaks at two or more, e.g. three or more, or four or more, and in particular five diffraction angles (2θ) selected from 9.5°, 10.2°, 14.7°, 15.6° and 26.2° (±0.2°).
7.76 A substantially crystalline form of a compound of formula (18) which exhibits peaks at the diffraction angles set forth in Table A-7 provided above or Table 7 provided below which have a relative intensity of at least 15%.
7.77 A substantially crystalline form of a compound of formula (18) which exhibits peaks at the diffraction angles corresponding to those of the X-ray powder diffraction pattern shown in Figure 7.
7.78 A substantially crystalline form of a compound of formula (18) having an X-ray powder diffraction pattern substantially as shown in Figure 7.

Further aspects and embodiments of the invention will be apparent from the Examples provided below.

### BRIEF DESCRIPTION OF THE DRAWINGS

Figure 1 is a XRPD Pattern of a crystalline form of the intermediate of formula (12).
Figure 2 is a XRPD Pattern of a crystalline form of the intermediate of formula (13).
Figure 3 is a XRPD Pattern of a crystalline form of the intermediate of formula (14).
Figure 4 is a XRPD Pattern of a crystalline form of the intermediate of formula (15).
Figure 5 is a XRPD Pattern of a crystalline form of the intermediate of formula (17).
Figure 6 is a XRPD Pattern of a crystalline form of a benzenesulphonate salt of the intermediate of formula (18).
Figure 7 is a XRPD Pattern of a crystalline form of the free base of the intermediate of formula (18).

### EXAMPLES

The invention will now be illustrated, but not limited, by reference to the specific embodiments described in the following examples.

### Abbreviations

In the examples, the following abbreviations are used.
- AcOH: acetic acid
- aq: aqueous
- BSA: benzenesulfonic acid
- DAST: diethylaminosulfur trifluoride
- DCM: dichloromethane
- DIPEA: N,N-diisopropylethylamine
- DMSO: dimethylsulfoxide
- EtOAc: ethyl acetate
- EtOH: ethanol
- h: hour(s)
- HPLC: high performance liquid chromatography
- iPrOAc: isopropyl acetate
- iPrMgCl: isopropylmagnesium chloride
- KF: Karl Fisher
- LC: liquid chromatography
- LCMS: liquid chromatography-mass spectrometry
- MeCN: acetonitrile
- min: minute(s)
- MgSO₄: magnesium sulfate
- NMR: nuclear magnetic resonance
- RT: retention time
- THF: tetrahydrofuran

### Analytical Methods

### HPLC Method 1

HPLC analysis was carried out on an Agilent 1100 series HPLC system. The column used was an Aquity BEH Phenyl; 30 × 4.6mm, 1.7 µm particle size (Ex Waters, PN: 186004644). The flow rate was 2.0 mL/min. Mobile phase A was Water: Trifluoroacetic acid (100:0.03%) and mobile phase B was Acetonitrile : Trifluoroacetic acid (100:0.03%). Detection was by UV at 210 nm. The injection volume was 5 µL, column temperature 40 °C and the following gradient was used:

| Time | %A | %B |
|---|---|---|
| 0 | 95 | 5 |
| 5.2 | 5 | 95 |
| 5.7 | 5 | 95 |
| 5.8 | 95 | 5 |
| 6.2 | 95 | 5 |

### HPLC Method 2

HPLC analysis was carried out on an Agilent 1110 series HPLC system. The column used was an Aquity BEH Phenyl; 30 × 4.6mm, 1.7 µm particle size (Ex Waters, PN: 186004644). The flow rate was 2.0 mL/min. Mobile phase A was Water: Trifluoroacetic acid (100:0.03%) and mobile phase B was Acetonitrile : Trifluoroacetic acid (100:0.03%). Detection was by UV at 210 nm. The injection volume was 5 µL, column temperature 40 °C and the following gradient was used:

| **Time (min)** | **%A** | **%B** |
|---|---|---|
| 0 | 95 | 5 |
| 5 | 95 | 5 |
| 15 | 5 | 95 |
| 16 | 5 | 95 |
| 16.5 | 95 | 5 |
| 17 | 95 | 5 |

### HPLC Method 3

HPLC analysis was carried out on an Agilent 1100/1200 series liquid chromatograph. The column used was an XSelect Phenyl-Hexyl; 150 × 4.6mm, 2.5 µm particle size (Ex Waters, PN: 186006735). The flow rate was 1.0 mL/min. Mobile phase A was 10mM Ammonium Acetate (pH 5.8) and mobile phase B was Acetonitrile 100%. Detection was by UV at 302 nm. The injection volume was 5 µL and column temperature 50 °C and the following gradient was used:

| **Time (min)** | **%A** | **%B** |
|---|---|---|
| 0 | 95 | 5 |
| 20 | 5 | 95 |
| 24.5 | 5 | 95 |
| 25 | 95 | 5 |

### Proton-NMR

Structures of all intermediates were confirmed from their ¹H NMR spectra which were collected using a JEOL ECX 400MHz spectrometer equipped with an auto-sampler. The samples were dissolved in a suitable deuterated solvent for analysis. The data was acquired using Delta NMR Processing and Control Software version 4.3.

### X-Ray Powder Diffraction (XRPD)

X-Ray Powder Diffraction patterns were collected on a PANalytical diffractometer using Cu Kα radiation (45kV, 40mA), θ - θ goniometer, focusing mirror, divergence slit (1/2"), soller slits at both incident and divergent beam (4mm) and a PlXcel detector. The software used for data collection was X'Pert Data Collector, version 2.2f and the data was presented using X'Pert Data Viewer, version 1.2d. XRPD patterns were acquired under ambient conditions via a transmission foil sample stage (polyimide - Kapton, 12.7µm thickness film) under ambient conditions using a PANalytical X'Pert PRO. The data collection range was 2.994 - 35°2θ with a continuous scan speed of 0.202004°s-1.

### Example 1

### 5-[[5-[4-(4-Fluoro-1-methyl-4-piperidyl)-2-methoxy-phenyl]-1H-pyrazol-3-yl]amino]pyrazine-2-carbonitrile was prepared as outlined in the reaction scheme shown below.

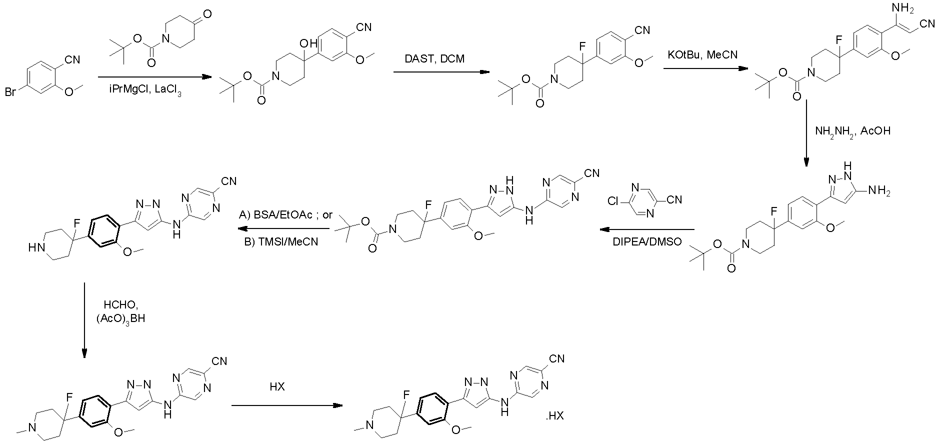

### Step 1: tert-Butyl 4-(4-cyano-3-methoxy-phenyl)-4-hydroxy-piperidine-1-carboxylate

To a 2 L vessel under nitrogen was charged 4-bromo-2-methoxybenzonitrile (125 g, 589 mmol) and anhydrous THF (375 mL). The slurry was cooled to 0°C. A 2M solution of iPrMgCl in THF (530 mL, 1.06 mol) was charged at 0-10°C over 20 min. The batch was stirred out at 0-5°C for 2 h. A 0.6M solution of LaCl₃.2LiCl in THF (196 mL, 118 mmol) was charged over 15 min at 0-5°C. After 30 min, a solution of N-Boc-4-piperidone (146.7 g, 736 mmol) in anhydrous THF (375 mL) was charged over 25 min at 0-15°C. After 30 min stirring, 10% AcOH (750 mL, 1.31 mol) was charged over 15 min at 0-30°C. The organic layer was separated off and concentrated to remove residual THF. The aqueous layer was extracted with TBME (750 mL) and combined with the concentrated organic layer. Water (250 mL) was charged, the batch stirred and the organic layer separated off and dried (MgSO₄). The batch was concentrated *in vacuo.* The crude material was dissolved in diisopropylether (500 mL) and transferred to a clean 2L vessel along with additional diisopropylether (125 mL). The batch was heated to 60°C and heptanes (500 mL) charged over 15 min at 55-60°C. The batch was cooled to 10°C over 5 h and then stirred overnight to give a slurry. Heptanes (375 mL) were charged, and the batch cooled to 0 °C for 30 min. The solids were filtered off and washed with an ice-cold mixture of diisopropylether (125 mL) and heptanes (125 mL). The material was dried at 40°C to afford the title compound as a white solid (126 g, 64% yield).

HPLC (Method 1) RT 2.97 min, 99.0%. ¹H NMR purity >95%.

¹H NMR (CDCl₃) δ 7.49 (d, *J =* 8.0 Hz, 1H), 7.16 (s, 1H), 7.04 (d, *J =* 6.4 Hz, 1H), 4.05 (br s, 2H), 3.93 (s, 3H), 3.89 (brs, 2H), 2.04 (s, 1H), 1.94 (brs, 2H), 1.67 (d, *J* = 12.8 Hz, 2H), 1.457 (s, 9H).

The XRPD Diffraction Pattern of the product is shown in Figure 1 and a list of XRPD peaks is shown in Table 1 below.

**Table 1**

| **Pos. [°2Th.]** | **Height [cts]** | **FWHM [°2Th.]** | **d-spacing [Å]** | **Rel. Int. [%]** |
|---|---|---|---|---|
| 5.6000 | 1735.69 | 0.0768 | 15.78189 | 21.76 |
| 8.4803 | 292.33 | 0.0768 | 10.42688 | 3.66 |
| 8.8402 | 201.55 | 0.0768 | 10.00328 | 2.53 |
| 10.5646 | 4320.08 | 0.0768 | 8.37400 | 54.16 |
| 11.3121 | 936.29 | 0.1023 | 7.82226 | 11.74 |
| 11.6533 | 594.49 | 0.0768 | 7.59402 | 7.45 |
| 12.3565 | 122.26 | 0.1023 | 7.16341 | 1.53 |
| 13.0852 | 3130.62 | 0.0768 | 6.76603 | 39.25 |
| 13.2823 | 7977.08 | 0.1023 | 6.66609 | 100.00 |
| 14.0532 | 2954.03 | 0.1023 | 6.30211 | 37.03 |
| 14.5131 | 2409.62 | 0.1279 | 6.10342 | 30.21 |
| 15.2683 | 1843.56 | 0.1279 | 5.80317 | 23.11 |
| 15.6991 | 525.46 | 0.1023 | 5.64489 | 6.59 |
| 16.6487 | 4864.57 | 0.1279 | 5.32502 | 60.98 |
| 17.0043 | 7918.32 | 0.1279 | 5.21443 | 99.26 |
| 17.4105 | 1874.34 | 0.1279 | 5.09370 | 23.50 |
| 17.7688 | 744.06 | 0.1279 | 4.99179 | 9.33 |
| 18.4513 | 203.57 | 0.1279 | 4.80864 | 2.55 |
| 18.9950 | 271.50 | 0.1023 | 4.67223 | 3.40 |
| 19.9165 | 4151.30 | 0.1279 | 4.45808 | 52.04 |
| 20.5065 | 1486.53 | 0.1023 | 4.33113 | 18.64 |
| 21.1127 | 3158.29 | 0.1279 | 4.20812 | 39.59 |
| 21.3170 | 1437.40 | 0.0768 | 4.16824 | 18.02 |
| 21.7063 | 653.99 | 0.1023 | 4.09436 | 8.20 |
| 22.0421 | 1189.78 | 0.1279 | 4.03274 | 14.92 |
| 22.4436 | 940.52 | 0.1535 | 3.96150 | 11.79 |
| 22.8199 | 488.42 | 0.1023 | 3.89701 | 6.12 |
| 23.1044 | 159.67 | 0.1535 | 3.84967 | 2.00 |
| 23.5132 | 264.26 | 0.1279 | 3.78366 | 3.31 |
| 24.4148 | 1857.74 | 0.1535 | 3.64593 | 23.29 |
| 24.6907 | 2508.89 | 0.1535 | 3.60581 | 31.45 |
| 25.0496 | 309.28 | 0.1791 | 3.55497 | 3.88 |
| 25.7063 | 1933.03 | 0.2303 | 3.46562 | 24.23 |
| 26.1593 | 391.11 | 0.1535 | 3.40662 | 4.90 |
| 26.4576 | 86.37 | 0.1023 | 3.36889 | 1.08 |
| 26.8516 | 160.52 | 0.1791 | 3.32034 | 2.01 |
| 27.2606 | 435.57 | 0.1791 | 3.27145 | 5.46 |
| 27.7590 | 318.75 | 0.1279 | 3.21383 | 4.00 |
| 28.4296 | 471.16 | 0.1535 | 3.13953 | 5.91 |
| 28.6924 | 469.86 | 0.1791 | 3.11138 | 5.89 |
| 29.3541 | 477.03 | 0.2814 | 3.04273 | 5.98 |
| 30.3127 | 416.40 | 0.1791 | 2.94865 | 5.22 |
| 30.9007 | 88.21 | 0.1535 | 2.89387 | 1.11 |
| 31.5317 | 736.32 | 0.2047 | 2.83738 | 9.23 |
| 32.1524 | 173.14 | 0.1023 | 2.78402 | 2.17 |
| 32.8573 | 113.25 | 0.1791 | 2.72588 | 1.42 |
| 33.3269 | 36.43 | 0.1535 | 2.68854 | 0.46 |
| 33.8229 | 506.29 | 0.2047 | 2.65024 | 6.35 |
| 34.5475 | 84.42 | 0.1535 | 2.59629 | 1.06 |

### Step 2: tert-Butyl 4-(4-cyano-3-methoxy-phenyl)-4-fluoro-piperidine-1-carboxylate

To a 2 L flask under nitrogen was charged DCM (1.2 L) and DAST (69.8 g, 133 mmol). The solution was cooled to -10°C. A solution of tert-butyl 4-(4-cyano-3-methoxyphenyl)-4-hydroxy-piperidine-1-carboxylate (120 g, 361 mmol) in DCM (0.24 L) was charged over 1 h at -10°C. After the addition was complete the reaction was warmed to 15-20°C for 1 h. To a 2nd vessel was charged potassium bicarbonate (240 g, 2.40 mol) and water (1 L). The batch was stirred at room temperature to give a solution. The reaction solution was transferred into the aq. potassium bicarbonate solution over 20 min (nitrogen transfer) at 15-25°C. DCM (50 mL) was used as a line rinse. The quenched batch was stirred for 15 min, and the pH checked (>7). The organic layer was separated off. The aqueous layer was extracted with DCM (240 mL), with water (150 mL) added to aid the separation. The organic layers were combined, dried (MgSO₄) and concentrated in vacuo. Diisopropylether (360 mL) was added to dissolve the crude material and concentrated in vacuo to give a white solid (114 g). To a portion of the crude material (100 g) was charged diisopropylether (400 mL) and heptanes (400 mL). The batch was heated to 70 °C to afford a solution, which was cooled to ambient temperature over 1 h and stirred overnight. The batch was filtered, and the solids washed with a mixture of diisopropylether (100 mL) and heptanes (100 mL) to afford 80 g. The material was purified further, diisopropylether (240 mL) and heptanes (240 mL) were added. The batch was heated to 70°C to afford a solution which was cooled to ambient temperature over 1 h. The batch was filtered, and the solids washed with a mixture of diisopropylether (80 mL) and heptanes (80 mL) to afford the title compound as a white solid (72.6 g, 69% yield). HPLC (method 2) RT 10.58 min, 99.2%. ¹H NMR purity >95%.

¹H NMR (CDCl₃) δ 7.54 (d, *J =* 8.8 Hz, 1H), 7.01 (s, 1H), 6.91 (d, *J* = 8.0 Hz, 1H), 4.13 (br s, 2H), 3.95 (s, 3H), 3.14 (brs, 2H), 2.00-1.91 (m, 4H), 1.28 (s, 9H).

The XRPD Diffraction Pattern of the product is shown in Figure 2 and a list of XRPD peaks is shown in Table 2 below.

**Table 2**

| **Pos. [°2Th.]** | **Height [cts]** | **FWHM [°2Th.]** | **d-spacing [Å]** | **Rel. Int. [%]** |
|---|---|---|---|---|
| 5.5759 | 250.24 | 0.3070 | 15.85005 | 4.90 |
| 7.5637 | 321.23 | 0.1023 | 11.68836 | 6.29 |
| 7.7929 | 345.44 | 0.1023 | 11.34503 | 6.76 |
| 8.5029 | 2545.21 | 0.1023 | 10.39922 | 49.80 |
| 8.8671 | 2621.51 | 0.1023 | 9.97295 | 51.30 |
| 9.8931 | 290.90 | 0.1023 | 8.94087 | 5.69 |
| 10.2574 | 791.90 | 0.0768 | 8.62410 | 15.50 |
| 10.7802 | 4447.72 | 0.1023 | 8.20702 | 87.03 |
| 11.2298 | 256.52 | 0.1023 | 7.87941 | 5.02 |
| 11.9615 | 1025.58 | 0.1023 | 7.39905 | 20.07 |
| 12.4050 | 1531.64 | 0.1023 | 7.13550 | 29.97 |
| 12.9069 | 2973.82 | 0.0768 | 6.85914 | 58.19 |
| 13.0954 | 1177.13 | 0.0512 | 6.76078 | 23.03 |
| 13.5236 | 3618.70 | 0.1023 | 6.54770 | 70.81 |
| 13.9781 | 4563.06 | 0.1023 | 6.33579 | 89.29 |
| 14.2608 | 2190.09 | 0.1023 | 6.21081 | 42.85 |
| 14.6423 | 474.92 | 0.0768 | 6.04984 | 9.29 |
| 14.8791 | 553.09 | 0.0768 | 5.95410 | 10.82 |
| 15.3807 | 1482.72 | 0.1023 | 5.76104 | 29.01 |
| 15.7382 | 4638.02 | 0.1279 | 5.63096 | 90.75 |
| 16.3321 | 1449.96 | 0.1791 | 5.42752 | 28.37 |
| 16.7161 | 2547.86 | 0.1023 | 5.30370 | 49.85 |
| 16.9480 | 990.51 | 0.0512 | 5.23164 | 19.38 |
| 17.2303 | 1839.29 | 0.1279 | 5.14654 | 35.99 |
| 17.7874 | 2089.46 | 0.1023 | 4.98661 | 40.88 |
| 18.1798 | 668.68 | 0.1279 | 4.87985 | 13.08 |
| 18.6918 | 2219.86 | 0.1279 | 4.74732 | 43.44 |
| 18.8618 | 1893.05 | 0.1279 | 4.70490 | 37.04 |
| 19.2793 | 497.37 | 0.0768 | 4.60395 | 9.73 |
| 19.4832 | 937.67 | 0.1279 | 4.55623 | 18.35 |
| 19.9611 | 751.31 | 0.1023 | 4.44820 | 14.70 |
| 20.2790 | 900.98 | 0.1535 | 4.37920 | 17.63 |
| 20.7647 | 1357.93 | 0.1279 | 4.27785 | 26.57 |
| 21.0645 | 1929.94 | 0.0768 | 4.21764 | 37.76 |
| 21.7471 | 5110.63 | 0.1279 | 4.08677 | 100.00 |
| 22.1444 | 252.94 | 0.1535 | 4.01434 | 4.95 |
| 22.6313 | 1101.76 | 0.1535 | 3.92906 | 21.56 |
| 23.1518 | 1724.31 | 0.2047 | 3.84189 | 33.74 |
| 23.8098 | 608.11 | 0.1535 | 3.73720 | 11.90 |
| 24.3171 | 813.83 | 0.1535 | 3.66036 | 15.92 |
| 24.5245 | 646.68 | 0.1023 | 3.62987 | 12.65 |
| 25.1723 | 668.59 | 0.1535 | 3.53791 | 13.08 |
| 25.5338 | 301.43 | 0.1279 | 3.48863 | 5.90 |
| 25.8660 | 765.04 | 0.1279 | 3.44459 | 14.97 |
| 26.4572 | 570.66 | 0.1023 | 3.36894 | 11.17 |
| 26.7806 | 622.82 | 0.1279 | 3.32898 | 12.19 |
| 27.3338 | 304.36 | 0.1279 | 3.26286 | 5.96 |
| 27.6556 | 424.70 | 0.1791 | 3.22562 | 8.31 |
| 28.0073 | 120.65 | 0.1279 | 3.18590 | 2.36 |
| 28.6144 | 358.98 | 0.1791 | 3.11968 | 7.02 |
| 29.0738 | 202.73 | 0.1535 | 3.07142 | 3.97 |
| 29.4243 | 290.93 | 0.1023 | 3.03563 | 5.69 |
| 30.0826 | 345.67 | 0.1535 | 2.97068 | 6.76 |
| 30.9128 | 96.16 | 0.2047 | 2.89277 | 1.88 |
| 31.8509 | 152.62 | 0.1535 | 2.80968 | 2.99 |
| 32.7872 | 63.21 | 0.2047 | 2.73155 | 1.24 |
| 33.2960 | 75.62 | 0.2047 | 2.69097 | 1.48 |
| 34.2242 | 231.14 | 0.2047 | 2.62007 | 4.52 |

### Step 3: tert-butyl 4-[4-[(Z)-1-amino-2-cyano-vinyl]-3-methoxy-phenyl]-4-fluoro-piperidine-1 -carboxylate

To a 2L vessel was charged tert-butyl 4-(4-cyano-3-methoxy-phenyl)-4-fluoro-piperidine-1-carboxylate (60.2 g, 180 mmol) and anhydrous THF (180.6 mL). The batch was stirred at 15-25°C to achieve a solution. Anhydrous MeCN (18.7 mL, 360 mmol) was charged. A 1M solution of potassium tert-butoxide in THF (540 mL, 540 mmol) was charged over 10 min at 15-25°C followed by a line rinse (THF 60 mL). The batch was then warmed to 40°C for 4 h. The batch was cooled to 15-25°C and water (16.3 mL, 900 mmol) charged. After 10 min, the batch was concentrated to ~1/4 volume on the rotavapor. The crude material was slurried in water (903 mL) for 30 min at 15-25°C and then filtered. The solids were washed with water (300 mL) and dried at 40°C to afford the title compound as a tan solid (65.0 g, 96% yield). HPLC (method 2): RT 10.46 min, 94.4% (plus 1.4% β- ketonitrile). ¹H NMR purity >95%.

¹H NMR (CDCl₃) δ 7.39 (d, *J =* 8.0 Hz, 1H), 7.02 (s, 1H), 6.91 (d, *J* = 7.2 Hz, 1H), 5.31 (brs, 2H), 4.19 (s, 1H), 4.15 (brs, 2H), 3.92 (s, 3H), 3.17 (brs, 2H), 1.98-1.92 (m, 4H), 1.50 (s, 9H)

The XRPD Diffraction Pattern of the product is shown in Figure 3 and a list of XRPD peaks is shown in Table 3 below.

**Table 3**

| **Pos. [°2Th.]** | **Height [cts]** | **FWHM [°2Th.]** | **d-spacing [Å]** | **Rel. Int. [%]** |
|---|---|---|---|---|
| 5.4689 | 240.23 | 0.3582 | 16.15972 | 1.94 |
| 6.9829 | 350.51 | 0.1023 | 12.65909 | 2.83 |
| 7.8336 | 6225.23 | 0.0768 | 11.28620 | 50.34 |
| 8.3646 | 316.68 | 0.1535 | 10.57091 | 2.56 |
| 10.6621 | 112.95 | 0.3070 | 8.29763 | 0.91 |
| 12.3627 | 78.59 | 0.2047 | 7.15979 | 0.64 |
| 13.4836 | 60.04 | 0.1791 | 6.56700 | 0.49 |
| 13.8994 | 643.56 | 0.0768 | 6.37149 | 5.20 |
| 14.0839 | 397.10 | 0.0512 | 6.28842 | 3.21 |
| 14.6277 | 2289.66 | 0.1023 | 6.05586 | 18.52 |
| 15.0181 | 1309.61 | 0.1023 | 5.89932 | 10.59 |
| 15.4453 | 3272.62 | 0.1023 | 5.73708 | 26.47 |
| 15.7226 | 3017.13 | 0.1535 | 5.63651 | 24.40 |
| 16.5070 | 378.31 | 0.1023 | 5.37040 | 3.06 |
| 16.8754 | 1343.83 | 0.1535 | 5.25397 | 10.87 |
| 17.3024 | 1331.68 | 0.1279 | 5.12528 | 10.77 |
| 18.1709 | 526.77 | 0.1023 | 4.88221 | 4.26 |
| 18.5597 | 1711.79 | 0.0512 | 4.78081 | 13.84 |
| 18.8857 | 12365.81 | 0.1279 | 4.69902 | 100.00 |
| 19.4039 | 218.88 | 0.0768 | 4.57467 | 1.77 |
| 19.9053 | 47.78 | 0.1279 | 4.46055 | 0.39 |
| 20.7157 | 785.19 | 0.1023 | 4.28786 | 6.35 |
| 21.3288 | 1704.68 | 0.1279 | 4.16597 | 13.79 |
| 21.8478 | 1251.73 | 0.1279 | 4.06816 | 10.12 |
| 22.2264 | 1458.26 | 0.1535 | 3.99972 | 11.79 |
| 22.9060 | 453.68 | 0.1535 | 3.88256 | 3.67 |
| 23.8508 | 728.77 | 0.1279 | 3.73086 | 5.89 |
| 24.9169 | 1056.31 | 0.1023 | 3.57360 | 8.54 |
| 25.1239 | 1076.77 | 0.1279 | 3.54462 | 8.71 |
| 25.9638 | 51.11 | 0.1535 | 3.43183 | 0.41 |
| 26.8630 | 528.50 | 0.1535 | 3.31896 | 4.27 |
| 27.3926 | 395.22 | 0.1279 | 3.25599 | 3.20 |
| 28.1993 | 309.51 | 0.1279 | 3.16465 | 2.50 |
| 28.5628 | 638.11 | 0.1279 | 3.12519 | 5.16 |
| 29.0396 | 145.63 | 0.1535 | 3.07496 | 1.18 |
| 29.8128 | 764.20 | 0.2047 | 2.99695 | 6.18 |
| 30.0601 | 346.53 | 0.1023 | 2.97285 | 2.80 |
| 30.3923 | 252.10 | 0.1535 | 2.94111 | 2.04 |
| 30.7651 | 93.59 | 0.1535 | 2.90632 | 0.76 |
| 31.3153 | 74.69 | 0.1535 | 2.85650 | 0.60 |
| 31.6360 | 282.60 | 0.1535 | 2.82827 | 2.29 |
| 32.0261 | 405.16 | 0.1279 | 2.79471 | 3.28 |
| 32.8034 | 143.11 | 0.1279 | 2.73024 | 1.16 |
| 34.4285 | 200.93 | 0.2047 | 2.60500 | 1.62 |

### Step 4: tert-butyl 4-[4-(5-amino-1H-pyrazol-3-yl)-3-methoxy-phenyl]-4-fluoro-piperidine-1-carboxylate

To tert-butyl 4-[4-[(Z)-1-amino-2-cyano-vinyl]-3-methoxy-phenyl]-4-fluoro-piperidine-1-carboxylate (61.4 g, 163.6 mmol) was charged EtOH (246 mL) and the batch stirred at 15-25°C. Hydrazine monohydrate (9.6 mL, 196 mmol) was charged followed by glacial acetic acid (11.2 mL, 196 mmol). The batch was warmed to 75°C for 2 h. The batch was cooled to 15-25°C, and water (614 mL) charged. After 30 min stirring, the solids were filtered off and washed with water (2x246 mL). The material was dried at 50°C to afford the title compound an off-white solid (58.6 g, 91.7% yield). HPLC (method 1): 97.7% (1.6% amide). KF water measurement: 1.3%.

To reduce levels of the amide side product a portion of the material (49.4g) was slurried at 50°C with diisopropyl ether (250 mL) for 30 min and then isolated at 15-25°C. The solids were washed with diisopropyl ether (50 mL) and oven dried to afford 47.7g stage 4. HPLC (method): 0.8% amide. A portion of the material (41.5g) was dissolved in DCM (415 mL) at 15-25°C and washed with 1M K₂CO₃(2×100 mL). The organic layer was dried (MgSO4), concentrated and oven dried at 50°C to afford the title compound (39.7 g, 58% yield) as a pale-yellow solid. HPLC (method 3): RT 12.94 min, 96.4%. ¹H NMR purity >95%.

¹H NMR (CDCl₃) δ 10.35 (s, 1H), 7.54 (d, *J =* 7.6 Hz, 1H), 7.04 (s, 1H), 6.93 (d, *J* = 8.0 Hz, 1H), 5.99 (s, 1H), 4.16 (brs, 2H), 3.98 (s, 3H), 3.16 (brs, 2H), 3.16 (brs, 2H), 2.06-1.94 (m, 4H), 1.48 (s, 9H).

The XRPD Diffraction Pattern of the product is shown in Figure 4 and a list of XRPD peaks is shown in Table 4 below.

**Table 4**

| **Pos. [°2Th.]** | **Height [cts]** | **FWHM [°2Th.]** | **d-spacing [Å]** | **Rel. Int. [%]** |
|---|---|---|---|---|
| 5.4701 | 7934.94 | 0.1023 | 16.15636 | 100.00 |
| 9.0334 | 1038.47 | 0.1535 | 9.78967 | 13.09 |
| 11.0578 | 329.27 | 0.1023 | 8.00157 | 4.15 |
| 11.7201 | 725.22 | 0.1279 | 7.55088 | 9.14 |
| 12.4477 | 195.58 | 0.2047 | 7.11111 | 2.46 |
| 13.8066 | 261.35 | 0.1023 | 6.41411 | 3.29 |
| 14.4749 | 1757.29 | 0.1791 | 6.11945 | 22.15 |
| 15.4324 | 355.29 | 0.1791 | 5.74185 | 4.48 |
| 15.9245 | 437.99 | 0.1535 | 5.56549 | 5.52 |
| 16.3898 | 862.30 | 0.2558 | 5.40854 | 10.87 |
| 17.0079 | 6332.70 | 0.1791 | 5.21334 | 79.81 |
| 17.6039 | 1629.07 | 0.1535 | 5.03817 | 20.53 |
| 18.2161 | 517.84 | 0.1535 | 4.87020 | 6.53 |
| 18.9472 | 1746.24 | 0.1791 | 4.68389 | 22.01 |
| 19.3431 | 3115.50 | 0.2303 | 4.58891 | 39.26 |
| 20.9486 | 457.60 | 0.1791 | 4.24072 | 5.77 |
| 21.9689 | 245.25 | 0.1023 | 4.04602 | 3.09 |
| 22.6739 | 946.42 | 0.3070 | 3.92178 | 11.93 |
| 23.5183 | 1201.83 | 0.1535 | 3.78285 | 15.15 |
| 24.1304 | 458.22 | 0.2558 | 3.68826 | 5.77 |
| 25.7751 | 195.93 | 0.2558 | 3.45652 | 2.47 |
| 27.2189 | 933.74 | 0.2047 | 3.27637 | 11.77 |
| 28.7572 | 263.63 | 0.4093 | 3.10451 | 3.32 |
| 30.3511 | 41.55 | 0.6140 | 2.94501 | 0.52 |
| 31.4234 | 54.23 | 0.3070 | 2.84692 | 0.68 |
| 33.4134 | 66.59 | 0.4093 | 2.68178 | 0.84 |

### Step 5: tert-butyl 4-[4-[5-[(5-cyanopyrazin-2-yl)aminol-1H-pyrazol-3-yl]-3-methoxyphenyl]-4-fluoro-piperidine-1-carboxylate

To a 500 mL vessel under nitrogen was charged tert-butyl 4-[4-(5-amino-1H-pyrazol-3-yl)-3-methoxy-phenyl]-4-fluoro-piperidine-1-carboxylate (32.4 g, 83 mmol) followed by 5-chloro-2-cyanopyrazine (12.34 g, 91.3 mmol) and anhydrous DMSO (64.8 mL). DIPEA (18.1 ml, 103.8 mmol) was charged, and the batch warmed to 50°C for 20 h. The batch was cooled to ambient temperature and iPrOAc (162 mL) charged. The resulting solution was poured into water (324 mL) at 15-30°C, and the batch stirred for 1 h. The batch was filtered and washed with water (324 mL) and iPrOAc (162 mL). The solids were oven dried at 50°C to afford the title compound as a tan solid (39.6g, 94% yield). HPLC (method 3): RT 15.57 min, 97.6%. ¹H NMR purity >95%.

¹H NMR (DMSO-d6) δ 12.65 (s, 1H), 10.76 (s, 1H), 8.66 (s, 1H), 8.51 (brs, 1H), 7.67 (d, *J =* 8.0 Hz, 1H), 7.14 (s, 1H), 7.09 (d, *J =* 7.2 Hz, 1H), 4.01 (brs, 2H), 3.93 (s, 3H), 3.05 (brs, 2H), 2.13-1.88 (m, 4H), 1.42 (s, 9H).

The XRPD Diffraction Pattern of the product is shown in Figure 5 and a list of XRPD peaks is shown in Table 5 below.

**Table 5**

| **Pos. [°2Th.]** | **Height [cts]** | **FWHM [°2Th.]** | **d-spacing [Å]** | **Rel. Int. [%]** |
|---|---|---|---|---|
| 4.1954 | 569.49 | 0.0768 | 21.06200 | 7.12 |
| 5.3479 | 308.77 | 0.2558 | 16.52527 | 3.86 |
| 8.4656 | 363.88 | 0.1279 | 10.44499 | 4.55 |
| 8.7877 | 2144.83 | 0.1023 | 10.06290 | 26.83 |
| 9.4696 | 1383.59 | 0.1023 | 9.33971 | 17.31 |
| 11.7687 | 161.36 | 0.1279 | 7.51981 | 2.02 |
| 12.5013 | 2436.64 | 0.1023 | 7.08073 | 30.48 |
| 13.6052 | 1343.68 | 0.1279 | 6.50862 | 16.81 |
| 14.0353 | 243.89 | 0.1279 | 6.31012 | 3.05 |
| 14.9385 | 642.13 | 0.1279 | 5.93054 | 8.03 |
| 15.4436 | 3595.84 | 0.1279 | 5.73771 | 44.98 |
| 16.3066 | 7993.52 | 0.1279 | 5.43594 | 100.00 |
| 17.1845 | 911.03 | 0.1279 | 5.16015 | 11.40 |
| 17.7188 | 818.95 | 0.1023 | 5.00576 | 10.25 |
| 17.9227 | 471.10 | 0.0768 | 4.94926 | 5.89 |
| 18.1445 | 351.66 | 0.0768 | 4.88926 | 4.40 |
| 18.5853 | 1311.37 | 0.1279 | 4.77428 | 16.41 |
| 19.7149 | 578.53 | 0.1279 | 4.50319 | 7.24 |
| 20.2896 | 95.06 | 0.1535 | 4.37693 | 1.19 |
| 21.3211 | 567.64 | 0.1535 | 4.16744 | 7.10 |
| 22.0264 | 2078.15 | 0.1279 | 4.03558 | 26.00 |
| 22.3775 | 1697.27 | 0.1535 | 3.97304 | 21.23 |
| 23.3408 | 330.06 | 0.1279 | 3.81121 | 4.13 |
| 23.5470 | 528.13 | 0.1023 | 3.77831 | 6.61 |
| 23.7850 | 396.22 | 0.1023 | 3.74103 | 4.96 |
| 24.1442 | 509.60 | 0.1279 | 3.68618 | 6.38 |
| 24.9050 | 295.74 | 0.1023 | 3.57528 | 3.70 |
| 25.6568 | 660.72 | 0.1279 | 3.47219 | 8.27 |
| 25.8774 | 678.62 | 0.1535 | 3.44308 | 8.49 |
| 26.8002 | 260.78 | 0.1535 | 3.32659 | 3.26 |
| 27.5412 | 451.11 | 0.1535 | 3.23876 | 5.64 |
| 28.9481 | 108.84 | 0.1535 | 3.08447 | 1.36 |
| 29.3280 | 100.05 | 0.1535 | 3.04538 | 1.25 |
| 30.3511 | 235.36 | 0.1279 | 2.94501 | 2.94 |
| 31.3973 | 62.73 | 0.5117 | 2.84923 | 0.78 |
| 32.5428 | 164.39 | 0.1279 | 2.75150 | 2.06 |
| 34.4863 | 17.53 | 0.2558 | 2.60076 | 0.22 |

### Step 6A: 5-[[3-[4-(4-fluoro-4-piperidyl)-2-methoxy-phenyl]-1H-pyrazol-5-yl]amino]pyrazine-2-carbonitrile

To a flask under nitrogen was charged tert-butyl 4-[4-[5-[(5-cyanopyrazin-2-yl)amino]-1H-pyrazol-3-yl]-3-methoxy-phenyl]-4-fluoro-piperidine-1-carboxylate (1 g, 2.03 mmol) and 1,4-dioxane (15 mL). The batch was heated to 80-85°C for 1 h then cooled to ambient and the solvent was removed *in vacuo.* To the crude material was charged EtOAc (30 mL) and the batch heated to 60°C. Benzenesulfonic acid (801 mg, 5.08 mmol) was charged, and the slurry heated at 60°C for 26 h. The batch was cooled to ambient and filtered. The solids were washed with EtOAc (10 mL) and oven dried at 50°C to afford the title compound as its besylate salt as a light brown solid (1.15 g, 84%). HPLC (method 3): RT 9.09 min, 97.9%. ¹H NMR: 41% BSA, 0.8% EtOAc, 58% title compound.

The XRPD Diffraction Pattern of the product is shown in Figure 6 and a list of XRPD peaks is shown in Table 6 below.

**Table 6**

| **Pos. [°2Th.]** | **Height [cts]** | **FWHM [°2Th.]** | **d-spacing [Å]** | **Rel. Int. [%]** |
|---|---|---|---|---|
| 4.2512 | 8202.52 | 0.1279 | 20.78537 | 100.00 |
| 5.4758 | 110.94 | 0.4093 | 16.13946 | 1.35 |
| 6.5407 | 5574.71 | 0.1279 | 13.51398 | 67.96 |
| 8.6054 | 3916.84 | 0.1279 | 10.27559 | 47.75 |
| 8.9584 | 1846.09 | 0.1279 | 9.87150 | 22.51 |
| 10.1438 | 613.00 | 0.1279 | 8.72042 | 7.47 |
| 11.2947 | 167.75 | 0.1791 | 7.83431 | 2.05 |
| 12.4583 | 383.33 | 0.1279 | 7.10507 | 4.67 |
| 12.9796 | 700.07 | 0.1791 | 6.82084 | 8.53 |
| 14.5882 | 1324.20 | 0.1279 | 6.07217 | 16.14 |
| 15.2029 | 2717.18 | 0.2814 | 5.82799 | 33.13 |
| 16.2645 | 656.33 | 0.1279 | 5.44991 | 8.00 |
| 16.6296 | 534.95 | 0.1535 | 5.33109 | 6.52 |
| 17.0081 | 631.25 | 0.1279 | 5.21329 | 7.70 |
| 17.4344 | 246.19 | 0.1535 | 5.08675 | 3.00 |
| 18.2303 | 299.90 | 0.1791 | 4.86644 | 3.66 |
| 18.8886 | 1329.06 | 0.2558 | 4.69829 | 16.20 |
| 19.6313 | 441.99 | 0.1535 | 4.52220 | 5.39 |
| 19.8707 | 686.74 | 0.1023 | 4.46825 | 8.37 |
| 20.4588 | 2148.08 | 0.2303 | 4.34112 | 26.19 |
| 21.0567 | 337.15 | 0.2047 | 4.21919 | 4.11 |
| 21.7739 | 1025.60 | 0.1791 | 4.08180 | 12.50 |
| 22.2106 | 1157.29 | 0.2047 | 4.00252 | 14.11 |
| 22.6825 | 592.56 | 0.2047 | 3.92031 | 7.22 |
| 23.4872 | 498.04 | 0.2303 | 3.78779 | 6.07 |
| 24.4366 | 1367.64 | 0.2303 | 3.64273 | 16.67 |
| 25.5187 | 1290.15 | 0.2047 | 3.49066 | 15.73 |
| 26.7626 | 252.40 | 0.2047 | 3.33119 | 3.08 |
| 27.3332 | 764.66 | 0.2558 | 3.26293 | 9.32 |
| 29.1449 | 145.36 | 0.2558 | 3.06409 | 1.77 |
| 29.5113 | 139.30 | 0.2558 | 3.02688 | 1.70 |
| 30.2736 | 39.41 | 0.3070 | 2.95237 | 0.48 |
| 31.2593 | 222.46 | 0.2047 | 2.86148 | 2.71 |
| 33.5239 | 148.11 | 0.1791 | 2.67319 | 1.81 |

### Step 6B: 5-[[3-[4-(4-fluoro-4-piperidyl)-2-methoxy-phenyl]-1H-pyrazol-5-yl]amino]pyrazine-2-carbonitrile

As an alternative to Step 6A above, 5-[[3-[4-(4-fluoro-4-piperidyl)-2-methoxy-phenyl]-1H-pyrazol-5-yl]amino]pyrazine-2-carbonitrile was also prepared according to the following method.

To tert-butyl 4-[4-[5-[(5-cyanopyrazin-2-yl)amino]-1H-pyrazol-3-yl]-3-methoxy-phenyl]-4-fluoro-piperidine-1-carboxylate (1.0 g, 2.03 mmo) was added MeCN (10 mL) followed by iodotrimethylsilane (577 µl, 4.06 mmol) at 0-20°C. After 30 min 10% aqueous potassium carbonate (5 mL, 3.62 mmol) was charged (off-gassing observed) and the batch stirred for 30 min. The solids were then filtered off and washed with a mixture of MeCN (2 mL) and water (2 mL). The solids were oven dried at 50°C to afford the title compound as a light brown solid (766 mg, 96%). HPLC (method 3): RT 9.09 mun, 98.8%. ¹H NMR purity >95%.

¹H NMR (DMSO-d6) δ 12.65 (s, 1H), 10.50 (s, 1H), 8.66 (s, 1H), 8.51 (brs,1H), 7.67 (d, *J =* 8.4 Hz, 1H), 7.10 (s, 1H), 7.05 (d, *J =* 8.0 Hz, 1H), 3.92 (s, 3H), 3.48 (brs, 1H), 2.93-2.83 (m, 4H), 2.08-1.82 (m, 4H).

The XRPD Diffraction Pattern of the product is shown in Figure 7 and a list of XRPD peaks is shown in Table 7 below.

| **Pos. [°2Th.]** | **Height [cts]** | **FWHM [°2Th.]** | **d-spacing [Å]** | **Rel. Int. [%]** |
|---|---|---|---|---|
| 5.4686 | 133.02 | 0.3070 | 16.16070 | 23.89 |
| 7.2285 | 87.91 | 0.2047 | 12.22954 | 15.78 |
| 9.5118 | 475.92 | 0.1535 | 9.29834 | 85.46 |
| 10.2059 | 482.92 | 0.1279 | 8.66747 | 86.71 |
| 14.6692 | 481.13 | 0.2047 | 6.03884 | 86.39 |
| 15.6196 | 556.90 | 0.1279 | 5.67344 | 100.00 |
| 16.7239 | 82.50 | 0.5117 | 5.30122 | 14.81 |
| 18.1495 | 75.78 | 0.1535 | 4.88791 | 13.61 |
| 19.0095 | 84.49 | 0.4093 | 4.66868 | 15.17 |
| 20.0050 | 137.18 | 0.2303 | 4.43855 | 24.63 |
| 21.9383 | 205.92 | 0.1023 | 4.05158 | 36.98 |
| 22.6163 | 255.58 | 0.1279 | 3.93164 | 45.89 |
| 23.4429 | 70.03 | 0.2558 | 3.79484 | 12.58 |
| 24.0880 | 91.80 | 0.3070 | 3.69466 | 16.48 |
| 26.2023 | 373.13 | 0.6140 | 3.40113 | 67.00 |
| 26.9223 | 294.89 | 0.3070 | 3.31178 | 52.95 |
| 31.6514 | 54.38 | 0.4093 | 2.82693 | 9.76 |
| 34.1306 | 47.75 | 0.4093 | 2.62705 | 8.57 |

### Step 7: 5-[[3-[4-(4-fluoro-1-methyl-4-piperidyl)-2-methoxy-phenyl]-1H-pyrazol-5-yl]amino]pyrazine-2-carbonitrile

To 5-[[3-[4-(4-fluoro-4-piperidyl)-2-methoxy-phenyl]-1H-pyrazol-5-yl]amino]pyrazine-2-carbonitrile (60.79 g active, 155 mmol) was charged EtOH (608 mL). The batch was stirred at RT and 37% formalin (22.5 mL, 278 mmol) charged. Sodium triacetoxyborohydride (58.95 g, 278 mmol) was charged in four portions over 30 min at 15-25°C. After 2 h, an 8.5% ammonium hydroxide solution (608 mL) was charged over 20 min at <25°C. The batch was cooled to 0-5°C and filtered, washing the cake with water (2 × 304 mL). The wet material was returned to vessel with water (608 mL) and slurried at RT for 30 min. The batch was filtered and washed with water (304 mL). The solids were oven dried at 50°C to afford the title compound (51.4 g, 82% active yield (100% minus water and ethanol)).

HPLC (Method 3) RT 10.20 min, 97.0%. 1H NMR purity >95%.

1H NMR (DMSO-d6) δ 12.64 (s, 1H), 10.74 (s, 1H), 8.66 (s, 1H), 8.51 (brs,1H), 7.66 (d, *J =* 8.0 Hz, 1H), 7.12 (s, 1H), 7.07 (d, *J =* 8.8 Hz, 1H), 3.92 (s, 3H), 2.72-2.49 (m, 2H), 2.22-2.09 (m, 4H), 2.12 (s, 3H), 1.93-1.86 (m, 2H).

### Equivalents

The foregoing examples are presented for the purpose of illustrating the invention and should not be construed as imposing any limitation on the scope of the invention. It will readily be apparent that numerous modifications and alterations may be made to the specific embodiments of the invention described above and illustrated in the examples without departing from the principles underlying the invention. All such modifications and alterations are intended to be embraced by this application.

### EMBODIMENTS

In certain embodiments described below, the invention provides:
8.1. A process for the preparation of a compound of formula (15): 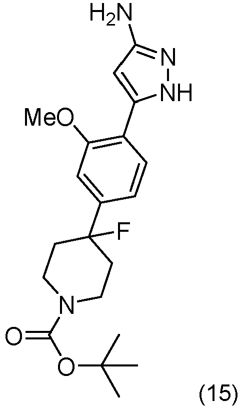
which process comprises reacting a compound of the formula (14): 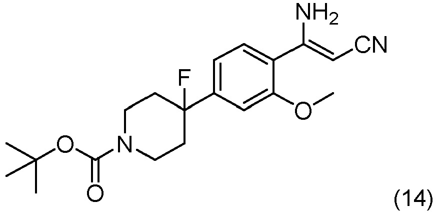
with hydrazine (NH₂NH₂).
8.2. A process for the preparation of a compound of the formula (14): 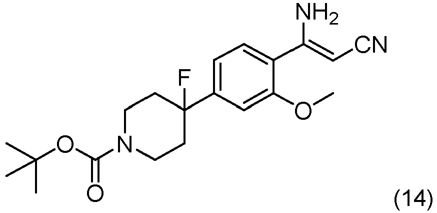
which process comprises reacting a compound of formula (13): 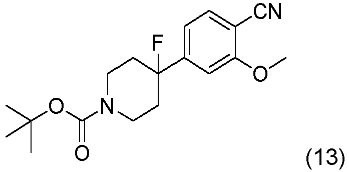
with acetonitrile (CH₃CN) in the presence of a base.
8.3. A process for the preparation of a compound of the formula (13): 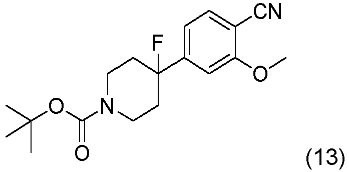
which process comprises reacting a compound of formula (12): 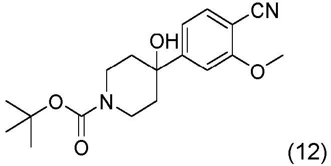
with a deoxyfluorination reagent.
8.4. A process for the preparation of a compound of the formula (12): 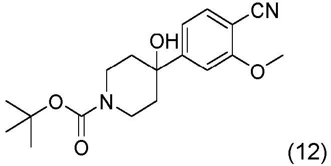
which process comprises reacting a compound of formula (10): 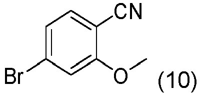
with a compound of formula (11): 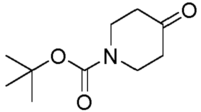
in the presence of a metallating agent, such as a Grignard reagent.
8.5. A process for the preparation of a compound of the formula (18): 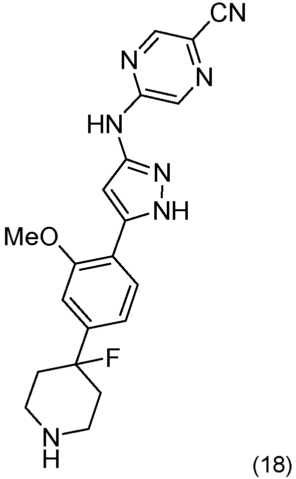
which process comprises reacting a compound of the formula (17): 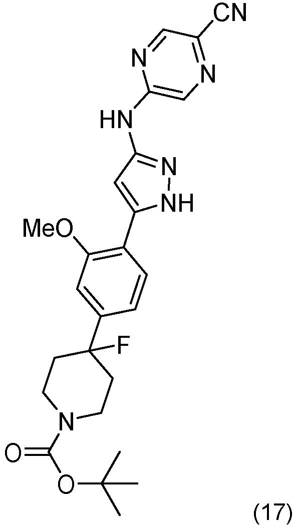
with an alkylsilyl halide or a sulphonic acid.
8.6. A process according to Embodiment 8.5 which process comprises reacting a compound of the formula (17) with an alkylsilyl halide (such as TMSI).
8.7. A process according to Embodiment 8.5 which process comprises reacting a compound of the formula (17) with a sulphonic acid (such as benzene sulphonic acid).
8.8. A process for the preparation of a compound of the formula (17): 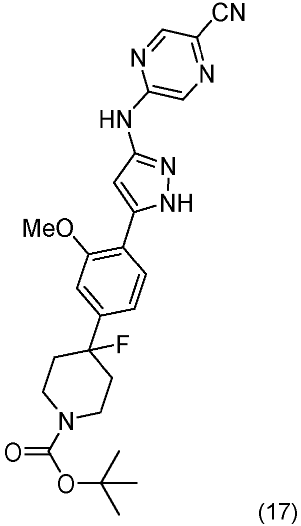
which process comprises the reaction of a compound of the formula (15) 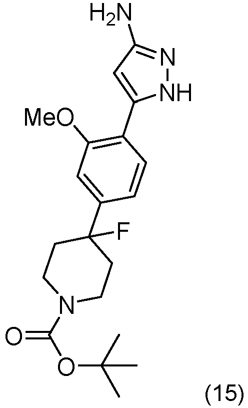
with a compound of the formula (16): 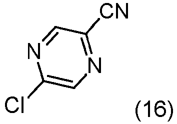
8.9. A process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
   i)
      a) a process according to Embodiment 8.4; and/or
      b) a process according to Embodiment 8.3; and/or
      c) a process according to Embodiment 8.2; and/or
      d) a process according to Embodiment 8.1; and/or
      e) a process according to Embodiment 8.8; and/or
      f) a process according to any one of Embodiments 8.5 to 8.7; and
   ii) interconverting a product obtained from step i) into 5-[[5-[4-(4-fluoro-1-methyl-4-piperidyl)-2-methoxy-phenyl]-1H-pyrazol-3-yl]amino]pyrazine-2-carbonitrile, for example by reacting a compound of formula (18) with a methylating agent (such as HCHO in the presence of a reducing agentsuch as (AcO₃)BH); and
   iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.
8.10. A process for the preparation of 5-[[5-[4-(4-fluoro-1-methyl-4-piperidyl)-2-methoxy-phenyl]-1H-pyrazol-3-yl]amino]pyrazine-2-carbonitrile, which process comprises:
   i)
      a) a process according to Embodiment 8.4; and/or
      b) a process according to Embodiment 8.3; and/or
      c) a process according to Embodiment 8.2; and/or
      d) a process according to Embodiment 8.1;
   ii) interconverting a product obtained from step i) to a compound of formula (18);
   iii) interconverting a product obtained from step ii) into 5-[[5-[4-(4-fluoro-1-methyl-4-piperidyl)-2-methoxy-phenyl]-1H-pyrazol-3-yl]amino]pyrazine-2-carbonitrile, for example by reacting a compound of formula (18) with a methylating agent (such as HCHO in the presence of a reducing agentsuch as (AcO₃)BH); and
   iv) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.
8.11. A compound of the formula (14): 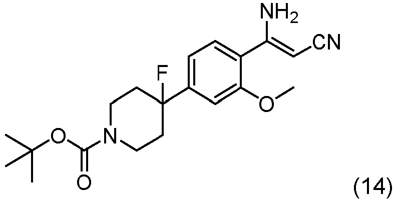
8.12. A compound of the formula (13): 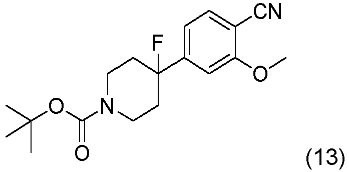
8.13. A compound of the formula (12): 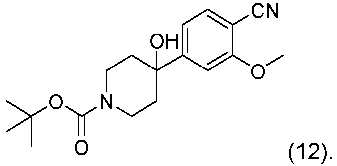
8.14. A compound according to any one of Embodiments 8.11 to 8.13 in a substantially crystalline form.
8.15. An invention as defined in any one of Embodiments 1.1 to 1.7, 2.1 to 2.6, 3.1 to 3.6, 4.1 to 4.8, 5.1 to 5.5, 6.1 to 6.8 and 7.1 to 7.78 herein.

## Claims

1. A process for the preparation of a compound of the formula (18): 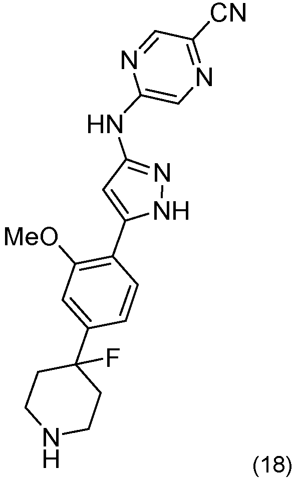 which process comprises reacting a compound of the formula (17): 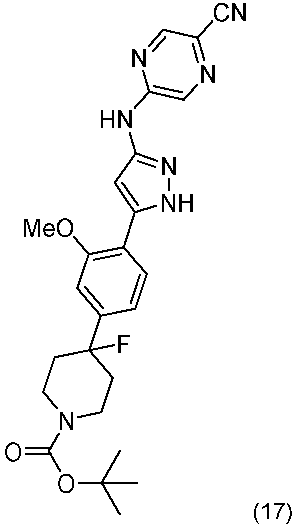 with an alkylsilyl halide or a sulphonic acid.

2. A process according to claim 1 which process comprises reacting a compound of the formula (17) with an alkylsilyl halide.

3. A process according to claim 2 wherein the alkylsilyl halide is trimethylsilyl iodide (TMSI).

4. A process according to any one of claims 1 to 3 wherein the reaction is carried out in the presence of a polar, aprotic solvent (such as acetonitrile).

5. A process according to any one of claims 1 to 4 wherein the reaction is carried out at a temperature of from 0 °C to 20 °C.

6. A process according to any one of claims 1 to 5 wherein the reaction is carried out for a period of from 15 minutes to 60 minutes, for example for a period of approximately 30 minutes and is optionally followed by the addition of a base (such as an alkali metal carbonate, e.g. potassium carbonate).

7. A process according to claim 1 which process comprises reacting a compound of the formula (17) with a sulphonic acid.

8. A process according to claim 7 wherein the sulphonic acid isbenzene sulphonic acid.

9. A process according to claim 7 or claim 8 wherein the reaction is carried out in the presence of a polar, aprotic solvent (such as ethyl acetate).

10. A process according to any one of claims 7 to 9 wherein the reaction is carried out at a temperature of from 50 °C to 70 °C, for example at a temperature of approximately 60 °C.

11. A process according to any one of claims 7 to 10 wherein the reaction is carried out for a period of from 20 hours to 30 hours, for example for a period of approximately 26 hours.

12. A process according to any one of claims 7 to 11 which, prior to reacting the compound of formula (17) with benzenesulphonic acid, comprises the step of reducing the water content of the starting material.

13. A process according to claim 12 wherein the step of reducing the water content of the starting material involves heating the compound of formula (17) in a non-polar solvent (such as 1,4-dioxane), for example at a temperature of from 80 °C to 85 °C, and then removing the non-polar solvent.

14. A process for the preparation of 5-[[5-[4-(4-fluoro-1-methyl-4-piperidyl)-2-methoxy-phenyl]-1 H-pyrazol-3-yl]amino]pyrazine-2-carbonitrile, which process comprises:
i) a process according to any one of claims 1 to 13; and
ii) interconverting a product obtained from step i) into 5-[[5-[4-(4-fluoro-1-methyl-4-piperidyl)-2-methoxy-phenyl]-1H-pyrazol-3-yl]amino]pyrazine-2-carbonitrile; and
iii) optionally forming a pharmaceutically acceptable salt of 5-[[5-[4-(4-fluoro-1-methyl-4-piperidyl)-2-methoxy-phenyl]-1H-pyrazol-3-yl]amino]pyrazine-2-carbonitrile.

15. A process according to claim 14 wherein the product obtained from step ii) is interconverted into 5-[[5-[4-(4-fluoro-1-methyl-4-piperidyl)-2-methoxy-phenyl]-1 Hpyrazol-3-yl]amino]pyrazine-2-carbonitrile by reacting the compound of formula (18) with a methylating agent (such as HCHO in the presence of a reducing agent such as (ACO)₃BH).
